# EUROPEAN PATENT APPLICATION

(11) **EP 4 653 449 A1**
(43) Date of publication of application: **26.11.2025**
(21) Application number: 24753276.5
(22) Date of filing: 02.02.2024
(51) Int. Cl.: C07K 1/02, B32B 7/12, B32B 9/02, B32B 37/12, C07K 2/00, C07K 14/435, C08G 75/045, C08G 81/00, C08J 3/20, C08J 5/00, C08K 5/37, C08L 89/02, C08L 101/16, C09D 189/00, C09D 189/02, C09J 189/00, C09J 189/02

(54) **METHOD FOR PRODUCING BLOCK COPOLYMER OR AQUEOUS DISPERSION CONTAINING SAME, ADHESIVE, COATING LIQUID, COATING AGENT, OR MOLDED BODY**

(30) Priority: 08.02.2023 JP 2023017760; 03.04.2023 JP 2023059942; 19.07.2023 JP 2023117420
(71) Applicant: Spiber Inc., Tsuruoka-shi, Yamagata 997-0052 (JP)
(72) Inventor: MUTO, Fumiya, Tsuruoka-shi, Yamagata 997-0052 (JP); SAKATA, Kazuki, Tsuruoka-shi, Yamagata 997-0052 (JP); KAGATA, Hideki, Tsuruoka-shi, Yamagata 997-0052 (JP); TAKAMI, Taku, Tsuruoka-shi, Yamagata 997-0052 (JP)
(74) Representative: Handley, Matthew Edward
(86) International application number: PCT/JP2024/003575
(87) International publication number: WO 2024/166833

(57) **Abstract**

The present invention provides a method for producing a block copolymer, the method including a step of mechanochemically treating a mixture containing a protein and a molecule capable of plasticizing a protein.

## Description

### Technical Field

The present invention relates to a method for producing a block copolymer or an aqueous dispersion containing the block copolymer, an adhesive, a coating liquid, a coating agent, or a molded body.

### Background Art

A structural protein such as a silk fibroin or a spider silk fibroin is attracting attention as an alternative to a structural material made of a synthetic resin and the like because the structural protein can exhibit excellent robustness. In recent years, development of recombinant technology has led to development of mass production technology of a recombinant structural protein imitating the above-described structural protein (for example, Patent Literature 1).

Generally, a protein has a structure in which a plurality of amino acids bind to each other, and a continuous structure of the amino acids is referred to as a main chain. An amide group (peptide bond) is present in a main chain of a protein, and many polar groups such as a carboxy group, an amide group, an amino group, a hydroxy group, and a thiol group are present in a side chain thereof. A protein retains its three-dimensional structure thereof by forming many intramolecular hydrogen bonds, and can change physical properties thereof by forming the intermolecular hydrogen bonds.

It is considered that a molded body such as a resin obtained by subjecting a structural protein to heat-and-pressure molding has excellent mechanical strength, but the molded body also has a brittle property in which cracking or rupture is caused by receiving an impact or the like. These circumstances are obstacles to using a protein as an alternative to a common general-purpose plastic.

It is also conceivable to improve flexibility by adding a plasticizer at the time of molding a structural protein as in technology of a general-purpose plastic, but since the plasticizer is generally hydrophobic, compatibility thereof with a protein having many polar groups is low. Therefore, even when a plasticizer is mixed with a structural protein, phase separation occurs between the structural protein and the plasticizer, and it is difficult to obtain an expected effect.

Therefore, the present inventor has found that, in a synthetic polymer (block copolymer) having a first segment including a polypeptide skeleton and one or more second segments binding to the first segment, in which the second segment includes a molecule having a plasticizing function with respect to the polypeptide skeleton, phase separation does not occur between a protein and a plasticizer, and a molded body having excellent flexibility can be produced (see Patent Literature 2).

### Citation List

### Patent Literature

Patent Literature 1: WO 2015/178466 A
Patent Literature 2: WO 2021/187502 A
Patent Literature 3: WO 2019/194263 A

### Summary of Invention

### Technical Problem

As a result of further studies by the present inventors, even the block copolymer described in Patent Literature 2 is produced by dissolving a protein in an organic solvent, and therefore an organic solvent that can be used is limited depending on the type of protein, and there is a possibility that the degree of freedom in reaction design is small. In addition, the organic solvent may remain in the produced block copolymer.

Therefore, an object of the present invention is to provide an advantageous method for producing a new block copolymer. Another object of the present invention is to provide an advantageous method for producing each of an aqueous dispersion, an adhesive, a coating liquid, a coating agent, and a molded body.

### Solution to Problem

The present inventor has found that a powdery block copolymer can be obtained by block-copolymerizing a protein under mechanochemical conditions. That is, the present invention provides the following [1] to [21].
[1] A method for producing a block copolymer, the method including a step of mechanochemically treating a mixture containing a protein and a molecule capable of plasticizing a protein.
[2] The method according to [1], wherein the block copolymer contains a protein and a molecule capable of plasticizing a protein as unit structures.
[3] The method according to [1] or [2], further including, after the step of performing the mechanochemical treatment, a step of freeze-drying the mechanochemically treated product.
[4] The method according to [1], wherein the number of reactive functional groups contained in the molecule capable of plasticizing a protein is larger than the number of reactive functional groups contained in the protein.
[5] The method according to any one of [1] to [4], wherein the use amount of the molecule capable of plasticizing a protein is larger than the use amount of the protein based on the number of moles.
[6] The method according to [5], wherein the reactive functional group in the protein is a nucleophilic functional group, and the reactive functional group in the molecule capable of plasticizing a protein is an electrophilic functional group.
[7] The method according to [6], wherein the nucleophilic functional group is a thiol group, and the electrophilic functional group is a thiol-reactive group.
[8] The method according to any one of [1] to [7], wherein the protein contains a hydrophobic protein.
[9] The method according to [8], wherein the hydrophobic protein has a hydropathy index of more than 0.
[10] The method according to any one of [1] to [9], wherein the protein contains an artificial protein.
[11] The method according to [10], wherein the artificial protein contains an artificial structural protein.
[12] The method according to any one of [1] to [11], wherein
   the protein contains two or more nucleophilic functional groups, and
   the molecule capable of plasticizing a protein contains two or more electrophilic functional groups.
[13] The method according to any one of [1] to [12], wherein
   the protein contains two or more thiol groups, and
   the molecule capable of plasticizing a protein contains two or more thiol-reactive groups.
[14] The method according to any one of [1] to [13], wherein a molecular weight of the molecule capable of plasticizing a protein is 10 or more when a molecular weight of the protein is 100.
[15] The method according to any one of [1] to [14], wherein a molecular weight of the molecule capable of plasticizing a protein is 100 or less when a molecular weight of the protein is 100.
[16] A method for producing a prepolymer, the method including a step of mechanochemically treating a mixture containing a protein and a molecule capable of plasticizing a protein.
[17] The method according to [16], wherein the block copolymer contains a protein and a molecule capable of plasticizing a protein as unit structures.
[18] The method according to [16] or [17], further including, after the step of performing the mechanochemical treatment, a step of freeze-drying the mechanochemically treated product.
[19] The method according to any one of [16] to [18], wherein the number of reactive functional groups contained in the molecule capable of plasticizing a protein is larger than the number of reactive functional groups contained in the protein.
[20] The method according to any one of [16] to [19], wherein the use amount of the molecule capable of plasticizing a protein is larger than the use amount of the protein based on the number of moles.
[21] The method according to [16] to [20], wherein the reactive functional group in the protein is a nucleophilic functional group, and the reactive functional group in the molecule capable of plasticizing a protein is an electrophilic functional group.
[22] The method according to [21], wherein the nucleophilic functional group is a thiol group, and the electrophilic functional group is a thiol-reactive group.
[23] The method according to any one of [16] to [22], wherein the prepolymer is powdery.
[24] A prepolymer containing a block copolymer containing a protein and a molecule capable of plasticizing the protein as unit structures.
[25] The prepolymer according to [24], which is powdery.
[26] An adhesive containing a block copolymer containing a protein and a molecule capable of plasticizing the protein as unit structures.
[27] The adhesive according to [26], which is powdery.
[28] A method for producing an aqueous dispersion of a block copolymer, the method including a step of dispersing a block copolymer obtained by the method according to any one of [1] to [15] in an aqueous medium.
[29] A method for producing a water-dispersible adhesive, the method including a step of dispersing a block copolymer obtained by the method according to any one of [1] to [15] in an aqueous medium.
[30] A method for producing a coating liquid, the method including a step of dispersing a block copolymer obtained by the method according to any one of [1] to [15] in an aqueous medium.
[31] A method for producing a molded body, the method including a step of molding a block copolymer obtained by the method according to any one of [1] to [15].
[32] The method according to [31], wherein the molding step is a step of subjecting the block copolymer to heat-and-pressure molding.
[33] A method for producing an aqueous dispersion of a block copolymer, the method including:
   a step of mechanochemically treating a mixture containing a protein and a molecule capable of plasticizing a protein to obtain a block copolymer; and
   a step of dispersing the obtained block copolymer in an aqueous medium.
[34] A method for producing a water-dispersible adhesive, the method including:
   a step of mechanochemically treating a mixture containing a protein and a molecule capable of plasticizing a protein to obtain a block copolymer; and
   a step of dispersing the obtained block copolymer in an aqueous medium.
[35] A method for producing a coating liquid, the method including:
   a step of mechanochemically treating a mixture containing a protein and a molecule capable of plasticizing a protein to obtain a block copolymer; and
   a step of dispersing the obtained block copolymer in an aqueous medium.
[36] A method for producing a molded body, the method including:
   a step of mechanochemically treating a mixture containing a protein and a molecule capable of plasticizing a protein to obtain a block copolymer; and
   a step of subjecting the obtained block copolymer to heat-and-pressure molding.
[37] The method according to [36], wherein the molding step is a step of subjecting the block copolymer to heat-and-pressure molding.
[38] A method for producing a solution-state adhesive, the method including a step of dissolving a block copolymer obtained by the method according to any one of [1] to [15] in a solvent.
[39] A method for producing a film-shaped adhesive, the method including a step of molding a film from a solution in which a block copolymer obtained by the method according to any one of [1] to [15] is dissolved.
[40] A method for producing a powdery adhesive, the method including a step of obtaining a powder composition containing a block copolymer obtained by the method according to any one of [1] to [15].
[41] A method for producing an adhesive body, the method including interposing a solution obtained by dissolving a block copolymer obtained by the method according to any one of [1] to [15] in a solvent between a plurality of adherends, and then removing the solvent from the solution to solidify the block copolymer, thereby bonding the adherends to each other.
[42] A method for producing an adhesive body, the method including interposing an aqueous dispersion obtained by dispersing a block copolymer obtained by the method according to any one of [1] to [15] in an aqueous medium between a plurality of adherends, and then removing the aqueous medium from the aqueous dispersion to solidify the block copolymer, thereby bonding the adherends to each other.
[43] A method for producing an adhesive body, the method including softening a film containing a block copolymer obtained by the method according to any one of [1] to [15] by swelling or heating, interposing the film between a plurality of adherends, and then curing the film in a state of being brought into pressure contact with the adherends, thereby bonding the adherends to each other.
[44] A method for producing an adhesive body, the method including heating a powder composition containing a block copolymer obtained by the method according to any one of [1] to [15] in a state where the powder composition is interposed between a plurality of adherends, and pressurizing the powder composition via the adherends to solidify the powder composition, thereby bonding the adherends to each other.
[45] A method for producing a solution-state coating agent, the method including a step of dissolving a block copolymer obtained by the method according to any one of [1] to [15] in a solvent.
[46] A method for producing a water-dispersible coating agent, the method including a step of dispersing a block copolymer obtained by the method according to any one of [1] to [15] in an aqueous medium.
[47] A method for producing a film-shaped coating agent, the method including a step of molding a film from a solution in which a block copolymer obtained by the method according to any one of [1] to [15] is dissolved.
[48] A method for producing a powdery coating agent, the method including a step of obtaining a powder composition containing a block copolymer obtained by the method according to any one of [1] to [15].
[49] A method for producing a laminate including a base material and a coating layer laminated on at least a part of a surface of the base material, the method including
   supplying a solution obtained by dissolving a block copolymer obtained by the method according to any one of [1] to [15] in a solvent to at least a part of the surface of the base material to coat at least a part of the surface of the base material with the solution, and then removing the solvent from the solution to solidify the block copolymer, thereby laminating the coating layer on at least a part of the surface of the base material.
[50] A method for producing a laminate including a base material and a coating layer laminated on at least a part of a surface of the base material, the method including
   supplying an aqueous dispersion obtained by dispersing a block copolymer obtained by the method according to any one of [1] to [15] in an aqueous medium to at least a part of the surface of the base material to coat at least a part of the surface of the base material with the aqueous dispersion, and then removing the aqueous medium from the aqueous dispersion to solidify the block copolymer, thereby laminating the coating layer on at least a part of the surface of the base material.
[51] A method for producing a laminate including a base material and a coating layer laminated on at least a part of a surface of the base material, the method including
   softening a film containing a block copolymer obtained by the method according to any one of [1] to [15] by swelling or heating, placing the film on at least a part of the surface of the base material, and then curing the film in a state of being brought into pressure contact with the base material, thereby laminating the coating layer on at least a part of the surface of the base material.
[52] A method for producing a laminate including a base material and a coating layer laminated on at least a part of a surface of the base material, the method including
   heating a powder composition containing a block copolymer obtained by the method according to any one of [1] to [15] in a state where the powder composition is placed on at least a part of the surface of the base material, and pressurizing the powder composition between a pressurizing body and the base material to solidify the powder composition, thereby laminating the coating layer on at least a part of the surface of the base material.

### Advantageous Effects of Invention

According to a method for producing a block copolymer according to the present invention, it is not necessary to dissolve a protein or a molecule capable of plasticizing a protein in an organic solvent. Therefore, not only wider types of proteins can be used as raw materials of a target block copolymer, but also simplification of production equipment can be advantageously achieved. Therefore, by the method according to the present invention, block copolymers having various structures can be produced more simply at low cost. Moreover, the residual amount of the organic solvent in the obtained block copolymer can be suppressed to zero or an extremely small amount (in a case of liquid assisted grinding). In addition, according to the present invention, an aqueous dispersion in which a block copolymer is more uniformly dispersed, an adhesive having excellent adhesive strength, a coating liquid capable of forming a coating film having a more uniform thickness, a coating agent capable of forming a coating layer having a more uniform thickness, and a molded body having excellent flexibility and strength can be obtained more simply at low cost.

### Brief Description of Drawings

Fig. 1 is a photograph illustrating appearances of powders and a film obtained in Comparative Example 1 and Example 1.
Fig. 2 is a graph illustrating results of GPC analysis of a film before and after reaction in Comparative Example 1.
Fig. 3 is a graph illustrating results of GPC analysis of a powder before and after reaction in Example 1.
Fig. 4 is a graph illustrating results of GPC analysis of a powder before and after reaction in Example 2.
Fig. 5 is a graph illustrating results of GPC analysis of powders before and after reaction in Examples 3 and 4.
Fig. 6 is a graph illustrating results of GPC of a powder prepared in Example 5.
Fig. 7 is a graph illustrating results of GPC of a powder prepared in Example 6.
Fig. 8(A) is a photograph illustrating a state in which products immediately before freeze-drying in Example 7 (left) and Comparative Example 2 (right) were dispersed in RO water. Fig. 8(B) is a photograph illustrating appearances of powders immediately after freeze-drying in Example 7 (left) and Comparative Example 2 (right). Fig. 8(C) is a photograph illustrating a state after a film obtained in Comparative Example 3 was pulverized. Fig. 8(D) is a photograph illustrating a state after a gel obtained in Comparative Example 4 was pulverized.
Fig. 9(A) illustrates particle sizes and occupancy ratios (accumulations) thereof in Example 7 (solid line) and Comparative Example 4 (broken line). Fig. 9(B) illustrates particle sizes and occupancy ratios thereof in Example 7 (solid line) and Comparative Example 4 (broken line).
Fig. 10(A) is a micrograph of a powder of Comparative Example 4, and Fig. 10(B) is a micrograph of a powder of Example 7.
Fig. 11 is a graph illustrating results of a turbidity test in Example 7 and Comparative Example 4.
Fig. 12(A) is a photograph illustrating a state of each of suspensions of Example 8 (left) and Comparative Example 5 (right). Fig. 12(B) is a photograph illustrating a state of each of the suspensions when each Eppendorf tube illustrated in Fig. 12(A) was placed upside down.
Fig. 13 is a photograph illustrating a state of each of coating films of Example 9 (left) and Comparative Example 6 (right).
Fig. 14(A) is a photograph illustrating a state of a resin film of Example 10, and Fig. 14(B) is a photograph illustrating a state of a resin film of Comparative Example 7.
Fig. 15(A) is a graph illustrating results of a tensile test, and Fig. 15(B) is a box plot illustrating results of elongation illustrated in Fig. 15(A).
Fig. 16 is a graph illustrating results of GPC of protein PRT2662 (broken line), Example 11A (dotted line), and Example 11B (solid line).
Figs. 17(A), 17(B), and 17(C) are photographs illustrating appearances of unmodified protein PRT2882, maleic acid esterified polyethylene glycol, and a product of Example 12, respectively.
Fig. 18 is a graph illustrating GPC chromatograms of unmodified protein PRT2882, maleic acid esterified polyethylene glycol, and a product of Example 12.
Fig. 19 is a graph illustrating GPC chromatograms of unmodified protein PRT3463, dialdehyde PEG, Example 13, and Comparative Example 8.
Fig. 20 is a photograph illustrating a state in which Example 13 and Comparative Example 8 were dissolved in DMSO.

### Description of Embodiments

Hereinafter, modes for carrying out the present disclosure will be described in detail with reference to the drawings in some cases. Note that the following embodiments are examples for describing the present disclosure, and are not intended to limit the present disclosure to the following contents.

Materials exemplified in the present specification can be used singly or in combination of two or more thereof unless otherwise specified. When a plurality of substances corresponding to each component in a composition is present, the content of each component in the composition means the total amount of the plurality of substances present in the composition unless otherwise specified.

### [Block copolymer]

A block copolymer according to the present embodiment is formed by binding a protein and a molecule capable of plasticizing a protein to each other. The block copolymer may have a repeating structure of a unit structure A composed of any one monomer of a protein and a molecule capable of plasticizing a protein and a unit structure B composed of the other monomer of these, or does not have to have a repeating structure of the unit structure A and the unit structure B. In addition, the number of each of the unit structures A and B contained in the block copolymer, a binding form thereof, and the like are not particularly limited. These are appropriately selected depending on properties and the like required for each of the block copolymer itself and a later-described composition or molded body obtained using the block copolymer. That is, the block copolymer may have, for example, a repeating structure such as an A-B diblock copolymer, an A-B-A triblock copolymer, an A-B-A-B tetrablock copolymer, or an A-B-A-B-A pentablock copolymer, or may have, for example, a non-repeating structure such as A-A-B-A-B. In addition, in the block copolymer according to the present embodiment, a binding position of the molecule capable of plasticizing a protein in a main chain of the protein is not limited at all. Such a binding position is appropriately selected, for example, according to properties and the like required for each of the block copolymer itself and a later-described composition or molded body obtained using the block copolymer. A preferable block copolymer has a structure having molecules capable of plasticizing a protein at both ends of the protein. Such a block copolymer is more suitable as a prepolymer because a protein easily reacts with a molecule capable of plasticizing a protein.

The block copolymer according to the present embodiment is formed by reaction (binding) between a reactive functional group (for example, a nucleophilic functional group) contained in a protein and a reactive functional group (for example, an electrophilic functional group) contained in a molecule capable of plasticizing a protein. In addition, a block copolymer containing a later-described modified protein into which a reactive functional group (for example, an electrophilic functional group) is introduced by chemical modification is formed by reaction (binding) between a reactive functional group (for example, an electrophilic functional group) of such a modified protein and a reactive functional group (for example, a nucleophilic functional group) of a molecule capable of plasticizing a protein.

Note that the number of reactive functional groups (for example, nucleophilic functional groups) contained in the protein and the number of reactive functional groups (for example, electrophilic functional groups) contained in the molecule capable of plasticizing a protein are not particularly limited, and can be appropriately changed depending on a structure, form, and the like of a target block copolymer. In other words, a block copolymer having a desired structure or form is formed by appropriately selecting the number of reactive functional groups contained in the protein or the number of reactive functional groups contained in the molecule capable of plasticizing a protein. For example, when the number of reactive functional groups contained in the protein and the number of reactive functional groups contained in the molecule capable of plasticizing a protein are the same, an obtained block copolymer can be formed as a finished polymer in which the reactive functional groups contained in the protein and the reactive functional groups of the molecule capable of plasticizing a protein have completely reacted with each other. In addition, a block copolymer in which the number of reactive functional groups contained in the molecule capable of plasticizing a protein is larger than the number of reactive functional groups contained in the protein can be formed as a prepolymer in which all of the reactive functional groups of the protein have reacted with the reactive functional groups of the molecule capable of plasticizing a protein, while the molecule capable of plasticizing a protein leaves reactive functional groups that have not reacted with the functional groups of the protein. Note that the number of reactive functional groups contained in the protein in the present specification refers to the number of reactive functional groups contained in one molecule of protein when such one molecule of protein is contained in the block copolymer, and refers to the total number of reactive functional groups contained in a plurality of molecules of protein when the plurality of molecules of protein are contained in the block copolymer. In addition, the number of reactive functional groups of the molecule capable of plasticizing a protein in the present specification refers to the number of reactive functional groups of one molecule capable of plasticizing a protein when such one molecule capable of plasticizing a protein is contained in the block copolymer, and refers to the total number of reactive functional groups of a plurality of types of molecules capable of plasticizing a protein in the block copolymer when the plurality of types of molecules capable of plasticizing a protein are contained in the block copolymer.

The position of the reactive functional group in the protein or the molecule capable of plasticizing a protein is not limited at all, and is appropriately selected according to a structure, properties, and the like required for the block copolymer. For example, the reactive functional groups may be located at both ends of the protein. The reactive functional groups located at both ends are likely to appear outside a conformation of the protein. Therefore, a protein having reactive functional groups at both ends easily reacts with a reactive functional group of a molecule capable of plasticizing a protein, and a block copolymer having a structure in which the protein and the molecule capable of plasticizing a protein linearly bind to each other is easily obtained. When the reactive functional groups are located at both ends of the protein, the molecules capable of plasticizing a protein can be located at both ends of the protein. Therefore, when the block copolymer is a prepolymer, the molecule capable of plasticizing a protein easily reacts with other compounds, and an advantage that formation of a network structure in a reaction using the prepolymer is easier can also be expected.

As described above, the block copolymer according to the present embodiment can have a desired structure or form by appropriately selecting the number or arrangement of reactive functional groups contained in the protein or the molecule capable of plasticizing a protein. For example, a block copolymer formed by binding a protein having a plurality of reactive functional groups and a molecule capable of plasticizing a protein and having a smaller number of reactive functional groups can have a structure formed by binding a plurality of molecules capable of plasticizing a protein per molecule of protein. On the other hand, a block copolymer formed by binding a molecule capable of plasticizing a protein and having a plurality of reactive functional groups and a protein having a smaller number of reactive functional groups can have a structure formed by binding a plurality of proteins per molecule capable of plasticizing a protein. In addition, for example, a block copolymer formed by binding a plurality of molecules of protein having reactive functional groups at both ends and a plurality of molecules capable of plasticizing a protein and having reactive functional groups at both ends can have the above-described repeating structure or non-repeating structure.

Note that, in the block copolymer according to the present embodiment, since a molecule capable of plasticizing a protein binds to a protein, a viscosity of the block copolymer in a molten state by a heating and pressurizing operation or a shearing operation can be reduced as compared with that of the protein based on its flexible properties. Furthermore, the block copolymer having a reduced viscosity is flowable, and a molded body can be formed by extrusion molding or the like.

The extrusion molding of the block copolymer according to the present embodiment does not necessarily require a plasticizer, but water and a polyhydric alcohol such as ethylene glycol, glycerol, or triglycerol may be used in order to further enhance fluidity.

The block copolymer according to the present embodiment can be produced by mechanochemically treating a mixture containing a protein and a molecule capable of plasticizing a protein.

### [Protein]

The protein may be a natural protein or an artificial protein. Alternatively, the protein may be a modified protein obtained by chemically modifying the natural protein or the artificial protein. Amino acid sequences of the natural protein, the artificial protein, and the modified protein are not particularly limited. In the present specification, the term "protein" includes a natural protein, an artificial protein, and a modified protein. Examples of the protein according to the present embodiment include a protein that can be used for industrial use and a protein that can be used for medical use. The phrase "can be used for industrial use" means, for example, that it can be used for various general-purpose materials used indoors or outdoors. Specific examples of the protein that can be used for industrial use include a structural protein. As the structural protein, for example, a protein having physical properties close to physical properties required for a desired application can be adopted. Specific examples of the structural protein include spider silk, silkworm silk, keratin, collagen, elastin, resilin, and proteins derived therefrom. A protein to be used may be an artificial fibroin or an artificial spider silk fibroin (artificial modified spider silk fibroin). Specific examples of the protein that can be used for medical use include an enzyme, a regulatory protein, a receptor, a peptide hormone, a cytokine, a membrane or a transport protein, an antigen used for vaccination, a vaccine, an antigen-binding protein, an immunostimulatory protein, an allergen, and a full length antibody or an antibody fragment or a derivative thereof.

In the present specification, "artificial protein" means a protein artificially produced, and includes a recombinant protein and a synthetic protein. The artificial protein may be a protein whose domain sequence is different from an amino acid sequence of a naturally derived protein. The "artificial protein" may be a protein in which an amino acid sequence is modified based on an amino acid sequence of a naturally derived protein (for example, a protein in which an amino acid sequence is modified by modifying a cloned gene sequence of a naturally derived protein), or a protein artificially designed and synthesized independently of a naturally derived protein (for example, a protein having a desired amino acid sequence by chemically synthesizing a nucleic acid encoding a designed amino acid sequence). Note that, in the artificial protein, an amino acid sequence can be freely designed unlike a natural protein. Therefore, when the artificial protein is used in a molding material or a molded body described later, a function, a property, a physical property, and the like of the molding material or the molded body can be arbitrarily controlled by appropriately designing the amino acid sequence of the artificial protein. In addition, since a uniform molecular design is always possible, a protein that has high homology with a target protein and is suitable for a purpose can be stably obtained. As a result, the quality a molding material or a molded body obtained using the artificial protein is advantageously stabilized. From this viewpoint, an artificial structural protein is advantageously used as the artificial protein.

The number of amino acid residues of the protein according to the present embodiment is not particularly limited, and may be, for example, 50 or more. In addition, the number of amino acid residues may be, for example, 100 or more, 150 or more, 200 or more, 250 or more, 300 or more, 350 or more, 400 or more, 450 or more, or 500 or more. The number of amino acid residues may be, for example, 5,000 or less, 4,500 or less, 4,000 or less, 3,500 or less, 3,000 or less, 2,500 or less, 2,000 or less, 1,500 or less, or 1,000 or less. As the number of amino acid residues is smaller, solubility in a solvent tends to increase. The preferable number of amino acid residues of the protein is, for example, 100 to 5000, 150 to 4500, 200 to 4000, 250 to 3500, 300 to 3000, 350 to 2500, 400 to 2000, 450 to 1500, or 500 to 1000.

A molecular weight of the protein according to the present embodiment is not particularly limited, and may be, for example, 2 kDa to 500 kDa. In addition, the molecular weight of the protein according to the present embodiment may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, and may be 500 kDa or less, 400 kDa or less, less than 360 kDa, 300 kDa or less, or 200 kDa or less. A preferable molecular weight of the protein is, for example, 2 kDa to 500 kDa, 3 kDa to 500 kDa, 4 kDa to 500 kDa, 5 kDa to 500 kDa, 6 kDa to 500 kDa, 7 kDa to 500 kDa, 8 kDa to 500 kDa, 9 kDa to 500 kDa, 10 kDa to 500 kDa, 20 kDa to 400 kDa, 30 kDa to 360 kDa, 40 kDa to 360 kDa, 50 kDa to 360 kDa, 60 kDa to 300 kDa, 70 kDa to 300 kDa, 80 kDa to 300 kDa, 90 kDa to 200 kDa, or 100 kDa to 200 kDa.

The protein according to the present embodiment contains at least one nucleophilic functional group such as a serine residue, a threonine residue, a tyrosine residue, a lysine residue, or a cysteine residue. In the modified protein, a hydroxy group of a serine residue, a threonine residue, or a tyrosine residue, an amino group of a lysine residue or the like, or a sulfhydryl group of a cysteine residue in the protein is acylated. By such acylation, an electrophilic functional group can be introduced into the modified protein. As an acylating agent used at this time, when an acylating agent having an electrophilic functional group such as a (meth)acrylic ester, a (meth)acrylamide, a maleic acid monoester, a maleic acid monoamide, a maleic acid diester, a maleic acid diamide, a maleimide, or an alkyl halide in the same molecule is used in addition to a site used in the acylation reaction, a reactive functional group can be introduced into the protein. When the protein has a serine residue, a threonine residue, or a tyrosine residue, the protein can be more efficiently acylated. In consideration of improvement in reactivity between the protein and the acylating agent, improvement in productivity of the block copolymer, and the like, the total content of a serine residue, a threonine residue, and a tyrosine residue in the protein (a ratio of the total number of serine residues, threonine residues, and tyrosine residues to the total number of amino acid residues) may be, for example, 4% or more, 4.5% or more, 5% or more, 5.5% or more, 6% or more, 6.5% or more, or 7% or more. Note that an upper limit value of the total content of a serine residue, a threonine residue, and a tyrosine residue is not particularly limited, but may be, for example, 35% or less, 33% or less, 30% or less, 25% or less, or 20% or less in consideration of amino acid compositions of various proteins that can be suitably used as the protein and the block copolymer according to the present embodiment.

As one aspect of the present embodiment, the protein may be a modified protein in which a nucleophilic functional group is introduced into a natural protein or an artificial protein. The nucleophilic functional group to be introduced may be, for example, a hydroxy group, an amino group, or a thiol group. The amino group may be a primary amino group (-NH₂) or a secondary amino group (-NHR in which R represents an alkyl group (for example, a C₁₋₆ alkyl group) or an alkenyl group (for example, a C₁₋₆ alkenyl group)). In this embodiment, since the protein has an electrophilic functional group as a reactive functional group, the molecule capable of plasticizing a protein preferably has a nucleophilic functional group as a reactive functional group. The block copolymer has a structure in which a nucleophilic functional group in a modified protein and an electrophilic functional group in a molecule capable of plasticizing the modified protein bind to each other. The block copolymer may have a repeating structure of a unit structure A composed of any one monomer of a protein and a molecule capable of plasticizing a protein and a unit structure B composed of the other monomer of these, or does not have to have a repeating structure of the unit structure A and the unit structure B. In addition, the number of each of the unit structures A and B contained in the block copolymer, a binding form thereof, and the like are not particularly limited. These are appropriately selected depending on properties and the like required for each of the block copolymer itself and a later-described composition or molded body obtained using the block copolymer. That is, the block copolymer may have, for example, a repeating structure such as an A-B diblock copolymer, an A-B-A triblock copolymer, an A-B-A-B tetrablock copolymer, or an A-B-A-B-A pentablock copolymer, or may have, for example, a non-repeating structure such as A-A-B-A-B. In addition, in the block copolymer according to the present embodiment, a binding position of the molecule capable of plasticizing a protein in a main chain of the protein is not limited at all. Such a binding position is appropriately selected, for example, according to properties and the like required for each of the block copolymer itself and a later-described composition or molded body obtained using the block copolymer. A preferable block copolymer has a structure having molecules capable of plasticizing a protein at both ends of the protein. Such a block copolymer is more suitable as a prepolymer because a protein easily reacts with a molecule capable of plasticizing a protein.

### [Hydrophobic protein]

The protein may be, for example, a hydrophobic protein. In a case where the protein is a hydrophobic protein, when a molded body is produced using a block copolymer as at least a part of a raw material, water resistance of the molded body is improved, and for example, when the molded body is used as a general-purpose material for industrial use, a life of the molded body can be advantageously extended. In addition, in the block copolymer, for example, when a functional substance is bound thereto, by controlling hydrophobicity or hydrophilicity of the functional substance, hydrophobicity or hydrophilicity of the entire conjugate of the functional substance and the block copolymer can be arbitrarily adjusted. When the protein has hydrophobicity (that is, when the protein is a hydrophobic protein), the entire conjugate can be shifted to a hydrophobic side as compared with a case where the protein has hydrophilicity, and thus hydrophobicity or hydrophilicity of the entire conjugate can be controlled over a wider range.

A hydropathy index of a protein can be estimated using an average HI value of amino acids constituting the protein as an index. In the present specification, the value of the average HI of a hydrophobic protein only needs to be more than 0, and may be, for example, 0.00 or more, 0.10 or more, 0.20 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. An upper limit value thereof is not particularly limited, and may be, for example, 1.00 or less or 0.7 or less.

Note that the value of the average HI of the hydrophobic protein and a hydropathy index of a repetitive sequence unit described later are determined according to a known method using a known hydrophobicity index of an amino acid residue. A known hydrophobicity index of an amino acid residue is presented in Table 1. For example, the hydropathy index may be calculated according to a method described in Kyte J & Doolittle R (1982) "A simple method for displaying the hydropathic character of a protein", J. Mol. Biol., 157, pp. 105 to 132.

**[Table 1]**

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

The hydrophobic protein preferably has low solubility in a lithium bromide aqueous solution (concentration: 9 M) at 60°C. That is, in a case where the hydrophobic protein is dissolved in a lithium bromide aqueous solution (concentration: 9 M) at 60°C, a maximum concentration may be, for example, less than 30% by mass, less than 25% by mass, less than 20% by mass, less than 15% by mass, less than 10% by mass, less than 5% by mass, or less than 1% by mass. Note that the hydrophobic protein may be completely insoluble in a lithium bromide aqueous solution (concentration: 9 M) at 60°C. When the hydrophobic protein has low solubility in a lithium bromide aqueous solution at 60°C, an obtained block copolymer easily acquires favorable water resistance (water resistance particularly suitable for industrial applications).

The hydrophobic protein preferably has a contact angle with water of 55° or more. The contact angle of the hydrophobic protein with water is more preferably 60° or more, 65° or more, or 70° or more. The contact angle with water can be evaluated by forming a membrane made of a hydrophobic protein on a base material, dropping water on the membrane, and measuring the contact angle after five seconds. When the contact angle of the hydrophobic protein with water is 55° or more, an obtained block copolymer easily acquires favorable water resistance (water resistance particularly suitable for industrial applications).

The hydrophobic protein preferably has excellent hot water resistance. Regarding the hot water resistance, for example, even when an aqueous dispersion of 5 w/v% of a hydrophobic protein is prepared and the dispersion is heated to 100°C, preferably, the protein is not decomposed for at least five hours. When the hydrophobic protein has excellent hot water resistance, an obtained block copolymer easily acquires favorable water resistance (water resistance particularly suitable for industrial applications) and hot water resistance.

### [Structural protein]

The protein according to the present embodiment may be, for example, a structural protein. The structural protein is a type of protein that can be used for industrial use, and means a protein related to a structure of a living body, a protein constituting a structure created by a living body, or a protein derived therefrom. The structural protein also refers to a protein that selfaggregates under predetermined conditions to form a structure such as a fiber, a film, a resin, a gel, a micelle, or a nanoparticle. Furthermore, the structural protein can also be said to be a protein in which a motif formed of a characteristic amino acid sequence or an amino acid number residue is repeatedly present to form a skeleton of an organism or a material. Examples of the structural protein in nature include fibroin, keratin, collagen, elastin, and resilin.

### [Artificial structural protein]

The structural protein may be an artificial structural protein. In the present specification, "artificial structural protein" means a structural protein artificially produced, and includes a synthetic protein and a recombinant structural protein produced from a microorganism by genetic recombination technology. The artificial structural protein may be a modified structural protein in which a part of an amino acid sequence of a naturally derived structural protein is modified depending on the amino acid sequence from a viewpoint of productivity, moldability, or the like.

The artificial structural protein according to the present embodiment may have a glycine residue content of 10 to 55% based on the number of amino acid residues. Note that, in the present specification, the "glycine residue content" is a value represented by the following formula. Glycine residue content = (Number of glycine residues contained in artificial structural protein/Number of all amino acid residues of polypeptide) × 100 (%)

The artificial structural protein may have 150 or more amino acid residues. The number of amino acid residues may be, for example, 200 or more or 250 or more and is preferably 300 or more, 350 or more, 400 or more, 450 or more, or 500 or more.

The artificial structural protein only needs to have a glycine residue content of 10 to 55% based on the number of amino acid residues. The glycine residue content may be, for example, 10% to 55%, 13% to 55%, 15% to 55%, 18% to 55%, 20% to 55%, 22% to 55%, or 25% to 55%.

In the artificial structural protein, the total content of the content of at least one type of amino acid residue selected from the group consisting of serine, threonine, and tyrosine (that is, any one of a serine residue content, a threonine residue content, a tyrosine residue content, the sum of the serine residue content and the threonine residue content, the sum of the serine residue content and the tyrosine residue content, the sum of the threonine residue content and the tyrosine residue content, and the sum of the serine residue content, the threonine residue content, and the tyrosine residue content), an alanine residue content, and a glycine residue content only needs to be 40% or more based on the number of amino acid residues. The total content may be, for example, 45% or more, 50% or more, 55% or more, or 60% or more. The upper limit of the total content is not particularly limited, and may be, for example, 90% or less, 85% or less, or 80% or less.

In one embodiment, in the artificial structural protein, the sum of a serine residue content, a threonine residue content, and a tyrosine residue content of 4% or more, 4.5% or more, 5% or more, 5.5% or more, 6% or more, 6.5% or more, or 7% or more based on the number of amino acid residues. The sum of the serine residue content, the threonine residue content, and the tyrosine residue content may be, for example, 35% or less, 33% or less, 30% or less, 25% or less, or 20% or less.

In the artificial structural protein according to the present embodiment, the distribution of serine residues, threonine residues, or tyrosine residues is mean, and the total content of the serine residue, threonine residue, and tyrosine residue in any consecutive 20 amino acid residues may be 4% or more, 5% or more, 10% or more, or 15% or more, and may be 50% or less, 40% or less, 30% or less, or 20% or less.

In addition, the alanine reside content, the serine residue content, the threonine residue content, and the tyrosine residue content have the same meaning as those obtained by replacing the alanine residue with a glycine residue, a serine residue, a threonine residue, and a tyrosine residue, respectively, in the above formula.

An artificial structural protein according to an embodiment may have a repetitive sequence. That is, the artificial structural protein according to the present embodiment may have a plurality of amino acid sequences (repetitive sequence units) having high sequence identity in the artificial structural protein. The number of amino acid residues of the repetitive sequence unit is preferably 6 to 200. The total number of glycine residues, serine residues, glutamine residues and alanine residues may be 40% or more, 45% or more, 50% or more, 55% or more, 60% or more, 65% or more, or 70% or more with respect to the total number of amino acid residues in the repetitive sequence unit. The sequence identity between the repetitive sequence units may be, for example, 80% or higher, 85% or higher, 90% or higher, 95% or higher, 96% or higher, 97% or higher, 98% or higher, or 99% or higher. A hydropathy index of the repetitive sequence unit (that is, a sum of HI values of amino acids contained in the repetitive sequence) may be, for example, -0.80 or more, -0.70 or more, -0.60 or more, -0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. Note that an upper limit value of the hydropathy index of the repetitive sequence unit is not particularly limited, and may be, for example, 1.0 or less or 0.7 or less.

An artificial structural protein according to an embodiment may include an (A)ₙ motif. In the present specification, the (A)ₙ motif means an amino acid sequence mainly containing an alanine residue. The number of amino acid residues in the (A)ₙ motif may be 2 to 27 and may be an integer of 2 to 20, 2 to 16, or 2 to 12. In addition, a ratio of the number of alanine residues to the total number of amino acid residues in the (A)ₙ motif only needs to be 40% or more, and may be 45% or more, 50% or more, 55% or more, 60% or more, 65% or more, 70% or more, 80% or more, 83% or more, 85% or more, 86% or more, 90% or more, 95% or more, or 100% (which means that the (A)n motif consists only of alanine residues). In the (A)ₙ motif, the total number of alanine residues, serine residues, threonine residues, and valine residues to the total number of amino acid residues in the (A)ₙ motif only needs to be 80% or more, but is preferably 85% or more, more preferably 90% or more, still more preferably 95% or more, and further still more preferably 100% (which means that the (A)n motif consists only of one or more amino acid residues selected from an alanine residue, a serine residue, a threonine residue, and a valine residue). A plurality of (A)ₙ motifs present in the recombinant structural protein according to the present embodiment may be the same amino acid sequence or different amino acid sequences. The (A)ₙ motif mainly contains alanine residues and tends to have α-helix structure or β-sheet structure. By inclusion of the (A)ₙ motif in the repetitive sequence unit, the artificial structural protein according to the present embodiment has these repetitive secondary structures. Therefore, as described later, when the artificial structural protein is in a form of a molded body such as a fiber, a film, or a resin, the artificial structural protein is expected to exhibit high strength due to these secondary structures.

Note that when the structural protein is molded, an amino acid having a relatively smaller side chain is more likely to form a hydrogen bond, and a molded body having higher strength is more likely to be obtained. In addition, an alanine residue and a glycine residue are amino acids having nonpolar side chains, and therefore are arranged so as to face inward in a folding process during polypeptide production and easily have an α-helix structure or a β-sheet structure. Therefore, a ratio of an amino acid such as a glycine residue or an alanine residue is desirably high. An alanine residue content only needs to be, for example, 10 to 40%, and may be 12 to 40%, 15 to 40%, 18 to 40%, 20 to 40%, or 22 to 40% from a viewpoint of obtaining a molded body having better strength. A glycine residue content only needs to be, for example, 10 to 55%, and may be 11 to 55%, 13 to 55%, 15 to 55%, 18 to 55%, 20 to 55%, 22 to 55%, or 25 to 55% from a viewpoint of obtaining a molded body having better strength.

In the present specification, the "alanine residue content" means the number of alanine residues to the total number of amino acid residues constituting a protein, and is a value represented by the following formula. Alanine residue content = (number of alanine residues contained in protein/total number of amino acid residues of protein) × 100 (%)

In addition, a glycine residue content, a serine residue content, a threonine residue content, a proline residue content, and a tyrosine residue content have the same meanings as those obtained by replacing the alanine residue with the glycine residue, the serine residue, the threonine residue, the proline residue, and the tyrosine residue, respectively, in the above formula.

In the structural protein, an amino acid having a relatively large side chain or an amino acid having flexibility is preferably homogenously contained in the entire sequence to a certain extent. Specifically, the structural protein may include a motif containing a tyrosine residue, a threonine residue, or a proline residue repeatedly in a cycle. When such a structural protein is used, formation of strong intermolecular hydrogen bonding is easily inhibited during processing of a molded body obtained by molding, and processability is easily improved. For example, the sum of a proline residue content, a threonine residue content, and a tyrosine residue content in any consecutive 20 amino acid residues may be 5% or more, more than 5.5%, 6.0% or more, more than 6.5%, 7.0% or more, more than 7.5%, 8.0% or more, more than 8.5%, 9.0% or more, 10.0% or more, or 15.0% or more. In addition, for example, the sum of a proline residue content, a threonine residue content, and a tyrosine residue content in any consecutive 20 amino acid residues may be 50% or less, 40% or less, 30% or less, or 20% or less.

In an artificial structural protein according to an embodiment, similarly to the protein described above, the sum of a serine residue content, a threonine residue content, and a tyrosine residue content may be, for example, 4% or more, 4.5% or more, 5% or more, 5.5% or more, 6% or more, 6.5% or more, or 7% or more from a viewpoint of improving reactivity with an acylating agent, improving productivity of a reaction-modified protein obtained by reaction with the acylating agent, or the like. The total content of a serine residue, a threonine residue, and a tyrosine residue may be, for example, 35% or less, 33% or less, 30% or less, 25% or less, or 20% or less.

The artificial structural protein may be an artificial fibroin. Examples of the fibroin include a naturally derived fibroin. Examples of the naturally derived fibroin include a fibroin produced by an insect or a spider. A natural fibroin is a fibrous protein, has a molecular weight of about 370,000, is constituted by two subunits, and has a high content of a glycine residue, an alanine residue, a serine residue, and a tyrosine residue, and these amino acid residues account for nearly 90% of the total number of amino acid residues. The natural fibroin has a crystalline region rich in amino acid residues having relatively small side chains, such as glycine, alanine, and serine, and an amorphous region having amino acid residues having relatively large side chains, such as tyrosine.

More specific examples of the naturally derived fibroin include a fibroin whose sequence information is registered in NCBI GenBank. For example, it can be confirmed by extracting a sequence in which spidroin, ampullate, fibroin, "silk and polypeptide", or "silk and protein" is described as a keyword in DEFINITION from sequences including INV as DIVISION among pieces of sequence information registered in NCBI GenBank, a sequence in which a specific character string of product is described from CDS, or a sequence in which a character string specific to TISSUE TYPE is described from SOURCE.

In the present specification, the "artificial fibroin" means an artificially produced fibroin (artificial fibroin). The artificial fibroin may be a fibroin having an amino acid sequence different from or identical to an amino acid sequence of a naturally derived fibroin. The artificial fibroin can be produced by a known method, for example, a method described in WO 2019/194263 A.

The artificial fibroin may be a fibrous protein having a structure similar to that of a naturally derived fibroin, or may be fibroin having a sequence similar to a repetitive sequence of the naturally derived fibroin. The "sequence similar to a repetitive sequence of the fibroin" may be an actual sequence of a naturally derived fibroin, or may be a sequence similar thereto.

The "artificial fibroin" may be a fibroin whose amino acid sequence is modified based on a naturally derived fibroin (for example, a fibroin whose amino acid sequence is modified by modifying a cloned gene sequence of a naturally derived fibroin) or a fibroin obtained by artificially designing an amino acid sequence independently of a naturally derived fibroin (for example, a fibroin having a desired amino acid sequence by chemically synthesizing a nucleic acid encoding a designed amino acid sequence) as long as the artificial fibroin has an amino acid sequence specified in the present disclosure. Note that a fibroin obtained by modifying an amino acid sequence of the artificial fibroin is also included in the artificial fibroin as long as an amino acid sequence thereof is different from an amino acid sequence of a naturally derived fibroin. Examples of the artificial fibroin include an artificial silk fibroin (a fibroin obtained by modifying an amino acid sequence of a silk protein produced by silkworms) and an artificial spider silk fibroin (a fibroin obtained by modifying an amino acid sequence of a spider silk protein produced by a spider). The artificial fibroin preferably contains an artificial spider silk fibroin as a molding material, and more preferably consists of the artificial spider silk fibroin because the artificial spider silk fibroin is relatively easily fibrillated and has a high fiber forming ability.

The artificial fibroin according to the present embodiment may be a protein including a domain sequence represented by Formula 1: [(A)ₙ motif-REP]ₘ or Formula 2: [(A)ₙ motif-REP]ₘ-(A)ₙ motif. An amino acid sequence (N-terminal sequence or C-terminal sequence) may be further added to any one or both of an N-terminal side and a C-terminal side of the domain sequence of the artificial fibroin. The N-terminal sequence and the C-terminal sequence are typically regions not containing repeats of amino acid motifs that are characteristic of a fibroin and each consist of an amino acid having about 100 residues, but are not limited thereto.

In the present specification, "domain sequence" is an amino acid sequence that generates a crystalline region (typically corresponding to an (A)ₙ motif of an amino acid sequence) and an amorphous region (typically corresponding to an REP of an amino acid sequence) specific to fibroin and is represented by Formula 1: [(A)ₙ motif-REP]ₘ or Formula 2: [(A)ₙ motif-REP]ₘ-(A)ₙ motif. Here, the (A)ₙ motif represents an amino acid sequence mainly consisting of alanine residues, and the number of amino acid residues is 2 to 27. The number of amino acid residues in the (A)ₙ motif may be an integer of 2 to 20, 4 to 27, 4 to 20, 8 to 20, 10 to 20, 4 to 16, 8 to 16, or 10 to 16. In addition, a ratio of the number of alanine residues to the total number of amino acid residues in the (A)ₙ motif is 40% or more and may also be 60% or more, 70% or more, 80% or more, 83% or more, 85% or more, 86% or more, 90% or more, 95% or more, or 100% (which means that the (A)ₙ motif consists of alanine residues). At least seven of a plurality of (A)ₙ motifs in the domain sequence may consist of alanine residues. The REP represents an amino acid sequence consisting of 2 to 200 amino acid residues. The REP may be an amino acid sequence consisting of 10 to 200 amino acid residues. m represents an integer of 2 to 300 and may be an integer of 10 to 300. The plurality of (A)ₙ motifs may be the same amino acid sequence or different amino acid sequences. The plurality of REPs may be the same amino acid sequence or different amino acid sequences.

Specific examples of the artificial fibroin include an artificial fibroin derived from a major dragline silk protein produced in a major ampullate gland of a spider as described in WO 2019/194263 A (first artificial fibroin), an artificial fibroin containing a domain sequence in which the content of glycine residues is reduced (second artificial fibroin), an artificial fibroin containing a domain sequence in which the content of an (A)ₙ motif is reduced (third artificial fibroin), an artificial fibroin in which the content of glycine residues and the content of an (A)ₙ motif are reduced (fourth artificial fibroin), an artificial fibroin containing a domain sequence including a region locally having a high hydropathy index (fifth artificial fibroin), and an artificial fibroin containing a domain sequence in which the content of glutamine residues is reduced (sixth artificial fibroin). Definition of each of the first to sixth artificial fibroins is incorporated herein by reference with the content described in WO 2019/194263 A.

The artificial fibroin may include a tag sequence at one or both of an N-terminal and a C-terminal thereof. This enables isolation, immobilization, detection, visualization, and the like of the artificial fibroin. PRT2662 having the amino acid sequence set forth in SEQ ID NO: 8 is a sixth artificial fibroin containing a tag sequence.

Examples of the tag sequence include an affinity tag using specific affinity (binding property or affinity) for another molecule. Specific examples of the affinity tag include a histidine tag (His tag). The His-tag is a short peptide in which about 4 to 10 histidine residues are lined up and can be used for isolating the artificial fibroin by chelating metal chromatography because the His-tag has a property of specifically binding to a metal ion such as nickel. Specific examples of the tag sequence include an amino acid sequence set forth in SEQ ID NO: 9 (an amino acid sequence including a His-tag sequence and a hinge sequence).

In addition, a tag sequence such as glutathione-S-transferase (GST) that specifically binds to glutathione or a maltose binding protein (MBP) that specifically binds to maltose can also be used.

Furthermore, an "epitope tag" using an antigenantibody reaction can also be used. By adding a peptide (epitope) exhibiting antigenicity as a tag sequence, an antibody to the epitope can be bound. Examples of the epitope tag include an HA (a peptide sequence of influenza virus hemagglutinin) tag, a myc tag, and a FLAG tag. By using the epitope tag, the artificial fibroin can be easily purified with high specificity.

Furthermore, a tag sequence that can be cleaved by a specific protease can also be used. By treating a protein adsorbed via the tag sequence with a protease, an artificial fibroin from which the tag sequence is cleaved can be collected.

Specific examples of the artificial fibroin include artificial fibroins presented in Table 2. Specific examples of the artificial fibroin also include PRT2662 (SEQ ID NO: 8), PRT2882 (SEQ ID NO: 10), and PRT3463 (SEQ ID NO: 11).

**[Table 2]**

| | Alanine residue content (%) | Glycine residue content (%) | Serine residue content (%) | Threonine residue content (%) | Tyrosine residue content (%) | Glutamine residue content (%) | Lysine residue content (%) |
|---|---|---|---|---|---|---|---|
| PRT799 (SEQ ID NO: 1) | 19.8 | 31.0 | 9.3 | 0 | 7.0 | 17.3 | 0.1 |
| PRT966 (SEQ ID NO: 2) | 19.7 | 31.2 | 9.4 | 0 | 7.1 | 0 | 0 |
| PRT918 (SEQ ID NO: 3) | 19.5 | 30.9 | 9.7 | 0 | 7.0 | 0 | 0 |
| PRT313 (SEQ ID NO: 4) | 25.7 | 36.0 | 8.9 | 0 | 6.4 | 8.2 | 0 |
| PRT1000 (SEQ ID NO: 5) | 19.4 | 30.8 | 9.6 | 0 | 7.0 | 0 | 0 |
| PRT1001 (SEQ ID NO: 6) | 19.3 | 30.7 | 9.6 | 0 | 6.9 | 0 | 0 |
| PRT587 (SEQ ID NO: 7) | 19.7 | 31.1 | 9.4 | 0 | 7.1 | 17.3 | 0 |

The artificial fibroin may be an artificial fibroin having at least two or more of the characteristics of the first artificial fibroin, the second artificial fibroin, the third artificial fibroin, the fourth artificial fibroin, the fifth artificial fibroin, and the sixth artificial fibroin.

A molecular weight of the artificial fibroin is not particularly limited, and may be, for example, 2 kDa or more and 700 kDa or less The molecular weight of the artificial fibroin according to the present embodiment may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, and may be 700 kDa or less, 600 kDa or less, 500 kDa or less, 400 kDa or less, less than 360 kDa, 300 kDa or less, or 200 kDa or less.

### [Production of protein by microbiological method]

The protein according to the present embodiment may be produced by a microbiological method. The protein may be produced with reference to description of WO 2017/188430 A, WO 2017/188434 A, WO 2017/222034 A, WO 2018/025886 A, WO 2019/022163 A, and the like.

The protein can be produced, for example, by a method including a step of expressing a nucleic acid in a host transformed with an expression vector. Examples of an expression method include not only direct expression but also secretory production and fusion protein expression according to a method described in Molecular Cloning, 2nd edition or the like. When expression is performed by yeast, an animal cell, or an insect cell, a protein can be obtained as a polypeptide to which a sugar or a sugar chain is added.

The protein according to the present embodiment can be produced by, for example, culturing the host transformed with the expression vector in a culture medium, producing and accumulating the protein in the culture medium, and collecting the protein from the culture medium. A method for culturing the host in the culture medium can be performed according to a method usually used for culturing a host.

### [Molecule capable of plasticizing protein]

The molecule capable of plasticizing a protein according to the present embodiment may contain a partial structure capable of plasticizing a protein and one or more reactive functional groups (for example, electrophilic functional groups). The molecule capable of plasticizing a protein can be a molecule in which an intermolecular force between the molecules is smaller than an intermolecular force between the proteins, and when the molecule capable of plasticizing a protein and the protein are mixed with each other, the molecule capable of plasticizing a protein can improve flexibility of a material or breaking elongation in bending or pulling as compared with the protein alone. Note that, as the molecule capable of plasticizing a protein, a molecule having biodegradability or a molecule derived from biomass is suitably used. Therefore, it is sufficiently expected that the block copolymer as a whole has biodegradability, and furthermore, production energy of the block copolymer can be reduced.

The molecule capable of plasticizing a protein forms a block copolymer by reaction of a reactive functional group therein with a reactive functional group contained in the protein. The molecule capable of plasticizing a protein and containing an electrophilic functional group as a reactive functional group reacts with a nucleophilic functional group as a reactive functional group contained in the protein to form a block copolymer. In addition, the molecule capable of plasticizing a protein and containing a nucleophilic functional group as a reactive functional group reacts with an electrophilic functional group as a reactive functional group introduced into a modified protein to form a block copolymer. As described above, the number of reactive functional groups contained in the molecule capable of plasticizing a protein and the positions thereof in the molecule capable of plasticizing a protein are not limited at all. The molecule capable of plasticizing a protein has at least one reactive functional group, preferably two or more reactive functional groups. The molecule capable of plasticizing a protein preferably has a chain-like chemical structure, and has one reactive functional group at each end of the molecular chain. Therefore, it can be expected to improve reactivity with a reactive functional group contained in a protein or with another compound.

Examples of the partial structure capable of plasticizing a protein include a polyether, a polyester, a polycarbonate, a polyamide, a polyol, a polyolefin, a polyacetal, a polyketal, a poly(meth)acrylate, silicone, a polyurethane, a polyalkyleneimine, a phenolic resin, a urea resin, and a melamine resin. Among these, a polyether, a polyester, a polycarbonate, a polyamide, a polyol, and the like are suitably used as the partial structure capable of plasticizing a protein, and a polyether, a polyester, a polycarbonate, and the like can be particularly suitably used.

Examples of the polyether include functional groups derived from polyalkylene glycols such as polyethylene glycol, polypropylene glycol, an ethylene oxide/propylene oxide copolymer, and polybutylene glycol. Note that, when these polyether groups are contained in the molecule capable of plasticizing a protein, the polyether may directly bind to a hetero element (O, N, or S) in an ester, a thioester, or an amide group of a later-described linker contained in the molecule capable of plasticizing a protein as necessary. Alternatively, the polyether may directly bind to a hetero element (O, N, or S) in an ester group, a thioester group, or an amide group of the protein. In this case, the entire polyether is easily separated from the linker or the protein, and as a result, a biodegradation rate of the polyether can be increased. A preferable partial structure is a polyalkylene glycol. Examples of the polyalkylene glycol include polyethylene glycol, polypropylene glycol, and polybutylene glycol.

When the partial structure capable of plasticizing a protein is a polyalkylene glycol, a molecular weight of the polyalkylene glycol moiety may be 1 kDa to 100 kDa, 2 kDa to 50 kDa, or 3 kDa to 25 kDa, and a molecular weight of the molecule capable of plasticizing a protein (soft segment) may be 100 Da to 500 kDa, 200 Da to 250 kDa, or 300 Da to 125 kDa.

Examples of the polyester include functional groups derived from polyesters such as polylactic acid, poly(3-hydroxybutanoic acid), a polyhydroxybutanoic acid/hydroxyvaleric acid copolymer, a polyhydroxybutanoic acid/4-hydroxybutanoic acid copolymer, a polyhydroxybutanoic acid/hydroxyhexanoic acid copolymer, polytrimethylene terephthalate, a butanediol/long-chain dicarboxylic acid copolymer, polyethylene terephthalate, polybutylene succinate, a polybutylene succinate adipate copolymer, a polybutylene adipate terephthalate copolymer, polycaprolactone, and poly(trimethylene furandicarboxylate) (PTF). Among the polyesters described above, the polyester group is preferably a functional group derived from being classified into a biomass plastic such as polycaprolactone or a biodegradable plastic.

Examples of the polycarbonate group include functional groups derived from polycarbonates having an aliphatic hydrocarbon chain as a main skeleton, such as 1,6-hexanediol polycarbonate, 1,5-pentanediol polycarbonate, and 1,10 decanediol carbonate.

Examples of the polyamide group include functional groups derived from polyamides such as nylon 3, nylon 4, nylon 5, nylon 6, nylon 11, and nylon 610. Among the polyamides described above, the polyamide group is preferably a functional group derived from being classified into a biomass plastic or a biodegradable plastic.

Examples of the polyol group (polyvinyl alcohol group or the like) include functional groups derived from polyols (polyvinyl alcohols and the like) such as polyvinyl alcohol and an ethylene-vinyl alcohol copolymer. Among the polyols described above, the polyol group is preferably a functional group derived from being classified into a biomass plastic or a biodegradable plastic.

Examples of the electrophilic functional group include a group reactive with a hydroxy group (hydroxy-reactive group), a group reactive with an amino group (amine-reactive group), and a group reactive with a thiol group (thiol-reactive group). Specific examples of the electrophilic functional group may include at least one structural unit represented by any one of formulas (1) to (20), may include at least one structural unit represented by any one of formulas (1), (2), (3), (4), (5), (6), (7), (8), (9), (10), (11), and (12), and from a viewpoint of improving biodegradability, may preferably include at least one structural unit represented by any one of formulas (1), (2), (3), (4), (5), and (12).

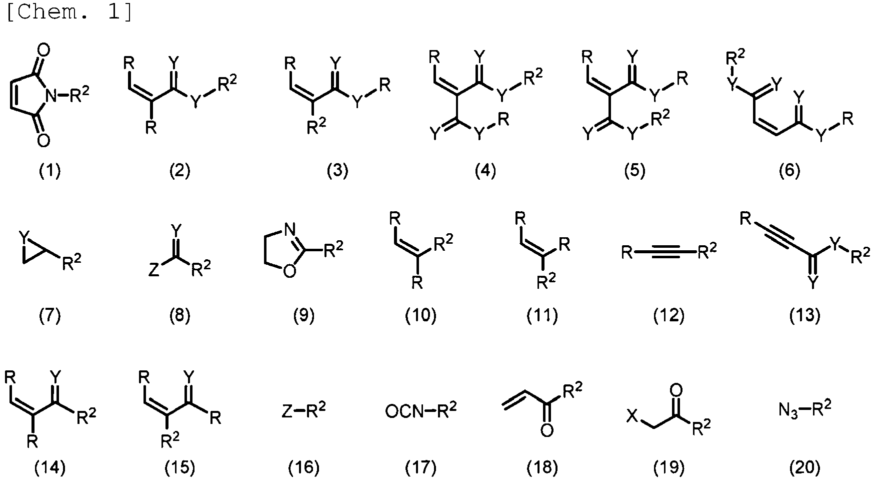

In formulas (1) to (20), R² represents the partial structure capable of plasticizing a protein. In formulas (2) to (8) and (13) to (15), Ys each independently represent an oxygen atom, a sulfur atom, or NR¹, and R¹ represents a hydrogen atom, a hydrocarbon group (for example, a C₁₋₆ alkyl group), an aromatic group (for example, a phenyl group, a naphthyl group, or a pyridyl group.), a carbonyl group, or a sulfonyl group. In formulas (2) to (6) and (10) to (15), Rs each independently represent a hydrogen atom, a hydrocarbon group (for example, a linear or branched C₁₋₆ alkyl group), or an aromatic group (for example, a phenyl group, a naphthyl group, or a pyridyl group.). In formulas (8) and (16), Z represents a hydrogen atom, a hydrocarbon group (for example, a C₁₋₆ alkyl group), an aromatic group (for example, a phenyl group, a naphthyl group, or a pyridyl group), a halogen atom (for example, a fluorine atom, a chlorine atom, a bromine atom, or an iodine atom), a sulfonic acid ester group, or a fluorine-containing carboxylic acid ester group. In general formula (19), X represents a halogen atom. The aromatic group may have a substituent of a halogen atom, a cyano group, a nitro group, an alkyl group (for example, a C₁₋₆ alkyl group), a haloalkyl group (for example, a trifluoromethyl group), or the like.

A non-limiting example of a chemical structure of the molecule capable of plasticizing a protein is described below. When the molecule capable of plasticizing a protein has, for example, one electrophilic functional group, the electrophilic functional group reacts with one nucleophilic functional group in the protein. When the molecule capable of plasticizing a protein has, for example, two or more electrophilic functional groups, a plurality of proteins can bind to one molecule of the molecule capable of plasticizing a protein by reaction of each of the electrophilic functional groups with a nucleophilic functional group contained in each of the plurality of molecules of the protein. In addition, when the molecule capable of plasticizing a protein has two or more electrophilic functional groups, the molecule capable of plasticizing a protein can bind to the protein while at least one of the plurality of electrophilic functional groups in the molecule capable of plasticizing a protein remains without reacting with a nucleophilic functional group in the protein. Such a polymer is suitable for use as a prepolymer.

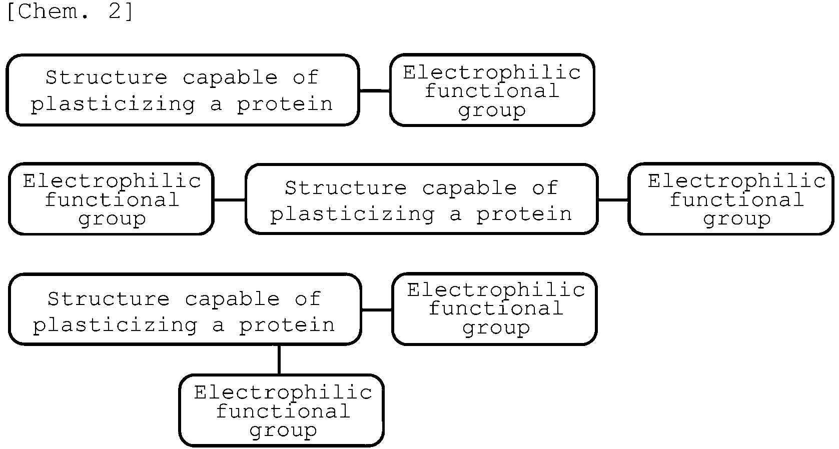

A preferable molecule capable of plasticizing a protein has one electrophilic functional group at each end of the structure capable of plasticizing a protein. A particularly preferable molecule capable of plasticizing a protein is a compound described below. In the formula, n may be 22 to 2273, 45 to 1136, or 68 to 568.

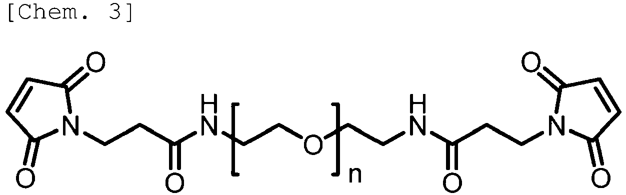

A molecular weight of the molecule capable of plasticizing a protein is, for example, 200 to 700,000, 250 to 500,000, 300 to 400,000, 350 to 350,000, 400 to 300,000, 500 to 200,000, 600 to 1,000,000, 700 to 50,000, 800 to 10,000, 900 to 7500, or 200 to 5000. When the molecular weight of the molecule capable of plasticizing a protein is 200 or more, the partial structure capable of plasticizing a protein is likely to be localized in a three-dimensional structure of a block copolymer. Therefore, it is not necessary to increase a weight ratio of the molecule capable of plasticizing a protein, which needs to be introduced in order to exhibit a certain level of function, more than necessary. As a result, it is not necessary to prolong a reaction time or to raise necessary reaction temperature in production of the block copolymer. On the other hand, when the molecular weight of the molecule capable of plasticizing a protein is 700,000 or less, binding reactivity of the molecule capable of plasticizing a protein to the protein is less likely to decrease. Note that the molecular weight of the molecule capable of plasticizing a protein is a weight average molecular weight, and is generally determined by a known method using GPC.

A ratio of the molecular weight of the molecule capable of plasticizing a protein to the protein can be appropriately adjusted according to an application of the block copolymer and the like. The molecular weight of the molecule (total molecular weight when two or more molecules capable of plasticizing a protein bind to one protein) may be, for example, 1 to 10,000, 1.5 to 9000, 2 to 8000, 3 to 7000, 5 to 5000, 7 to 3000, or 10 to 2000 based on the molecular weight 100 of the protein. When the molecular weight of the molecule with respect to the molecular weight of the protein is less than 1, plasticity (flexibility) may be insufficient in a molded body (for example, a fiber, a film, or a resin) obtained using the block copolymer. On the other hand, when the molecular weight of the molecule with respect to the molecular weight of the protein is more than 10,000, plasticity is excessively increased, and rigidity of the molded body may be reduced. Within the above range, it is possible to produce a molded body having better flexibility. In addition, the molecular weight of the molecule may be, for example, 1.0 or more, 1.2 or more, 1.3 or more, 1.4 or more, 1.5 or more, 1.6 or more, 1.7 or more, 1.8 or more, 1.9 or more, 2.0 or more, 5.0 or more, 10 or more, 20 or more, 30 or more, or 40 or more when the molecular weight of the protein is 100. Note that an upper limit value thereof is not particularly limited, and may be, for example, 200 or less, 150 or less, 100 or less, 80 or less, 70 or less, 60 or less, or 50 or less. Furthermore, the molecular weight of the molecule may be within a range of 1 to 200, or may be 1.5 to 150, 5 to 130, 10 to 100, or 10 to 70 when the molecular weight of the protein is 100. When the ratio between the molecular weight of the protein and the molecular weight of the molecule is a value within the above range, for example, in a molded body obtained using the block copolymer, it can be expected to enhance flexibility, stretchability, or the like of the entire block copolymer while sufficiently retaining properties (for example, high mechanical strength) due to having the protein. Note that the ratio of the molecular weight of the molecule when the molecular weight of the protein is 100 is determined as a weight average molecular weight.

The content ratio between the protein and the molecule in the block copolymer can be appropriately adjusted according to an application of the block copolymer and the like. As for such a content ratio, the protein may be, for example, 50 or more, 60 or more, 70 or more, 80 or more, 90 or more, 100 or more, 110 or more, 150 or more, 200 or more, 250 or more, 300 or more, 300 or more, 400 or more, 450 or more, 500 or more, 550 or more, or 600 or more when the molecule is 100 in terms of mass ratio. An upper limit value of such a value is not particularly limited, and may be 1,000 or less, 900 or less, 800 or less, or 700 or less. Since the content ratio between the protein and the molecule in the block copolymer is set to a value within the above range, wasteful use of the molecule can be suppressed, and production cost of the block copolymer can be suppressed. Note that reduction of the content ratio of the molecule to the protein in the block copolymer can be advantageously achieved by, for example, reducing the molecular weight of the protein.

The molecular weight of the protein is, for example, preferably 200 to 1,000,000, more preferably 300 to 900,000, still more preferably 400 to 800,000, still more preferably 500 to 700,000, still more preferably 600 to 600,000, still more preferably 1,000 to 600,000, still more preferably 3,000 to 600,000, still more preferably 5,000 to 600,000, still more preferably 10,000 to 600,000, and still more preferably 5,000 to 100,000. Note that when the molecular weight of the protein is less than 200, the protein (hard segment) may be too small with respect to the molecule capable of plasticizing a protein (soft segment). In this case, rigidity of a molded body to be molded using the block copolymer (molding material) is small, and there is a possibility that it is difficult to use the molded body, for example, as a structural body. On the other hand, when the molecular weight of the protein is more than 1,000,000, reactivity of a linking reaction is reduced. Therefore, the reaction cannot be completed in a time in which commercial production as a material can be performed in a chemically engineered manner. As a result, there is a possibility of leading to localization of unreacted molecules capable of plasticizing a protein remaining in the molded body. The molecular weight of the protein may be 1,000 or more, 2,000 or more, 3,000 or more, 4,000 or more, 5,000 or more, 6,000 or more, 7,000 or more, 8,000 or more, 9,000 or more, 10,000 or more, 20,000 or more, 30,000 or more, 40,000 or more, 50,000 or more, 60,000 or more, 70,000 or more, 80,000 or more, 90,000 or more, or 100,000 or more. Furthermore, the molecular weight of the protein may be 400,000 or less, less than 360,000, 300,000 or less, or 200,000 or less. When the molecular weight of the protein is 200,000 or less or 100,000 or less, it is expected that the use amount of the molecule capable of plasticizing a protein can be suppressed as much as possible in order to enhance flexibility of the block copolymer. Note that the molecular weight of the protein is a weight average molecular weight.

In the present specification, the molecular weight is a value measured by sodium dodecyl sulfatepolyacrylamide gel electrophoresis (SDS-PAGE). Note that the electrophoresis is performed in the following procedure. That is, first, 200 µL of a 2 M lithium chloride DMSO solution is added to 2 mg of a powder sample, and the mixture is stirred while being heated at 80°C for 60 minutes and further at 95°C for ten minutes to dissolve the sample. Thereafter, the sample is diluted to 50 times with a 10 M urea solution, further diluted to 2 times with a sample buffer (manufactured by FUJIFILM Wako Pure Chemical Corporation), and heated at 95°C for 5 minutes to denature the protein. Subsequently, a gel for SDS-PAGE (manufactured by Bio-lad) is attached to an electrophoresis device (manufactured by Bio-lad), the device is filled with an SDS buffer, and the electrophoresis device is connected to a power supply device (manufactured by Bio craft). 10 µL of the denatured sample is added to each well of the gel for SDS-PAGE, and a current of 30 mA/sheet is allowed to flow for 30 minutes. After completion of the electrophoresis, the gel for SDS-PAGE is taken out from the device, immersed in Oriole fluorescent gel stain (manufactured by Bio-lad), and shaken for one hour. Thereafter, the gel is placed on a UV sample tray (manufactured by Bio-lad), and a stained image is acquired with a GelDocEZ gel imaging device (manufactured by Bio-lad).

### [Method for producing block copolymer]

One embodiment of the present invention is a method for producing a block copolymer, the method including a step of mechanochemically treating a mixture containing a protein and a molecule capable of plasticizing a protein.

For a definition of the protein used in the method according to the present embodiment, the above-described definition of the protein can be taken into consideration. The protein preferably contains a hydrophobic protein. The hydrophobic protein preferably has a hydropathy index of more than 0. The protein may be an artificial protein, and preferably contains an artificial structural protein.

In a certain embodiment, for example, the protein has two or more nucleophilic functional groups, and the molecule capable of plasticizing a protein has two or more electrophilic functional groups. In this embodiment, the nucleophilic functional group and the electrophilic functional group bind to each other to form a block copolymer containing the protein and the molecule capable of plasticizing a protein as a monomer unit. In a preferable embodiment, the protein has two or more hydroxy groups, amino groups, or thiol groups, and the molecule capable of plasticizing a protein has two or more hydroxy-reactive groups, amine-reactive groups, or thiol-reactive groups.

The electrophilic functional group is, for example, a group represented by any one of formulas (1) to (20), a preferable electrophilic functional group is a group represented by formula (1), (2), (6) to (8), (16), or (19), and a more preferable electrophilic functional group is a group represented by formula (1), (2), or (6). Preferable combinations of a nucleophilic functional group and an electrophilic functional group are a combination of a hydroxy group and a hydroxy-reactive group represented by any one of formulas (2) to (9), (13) to (17), and (19), a combination of an amino group and an amine-reactive group represented by any one of formulas (2) to (9), (13) to (17), and (19), and a combination of a thiol group and a thiol-reactive group represented by any one of formulas (1) to (6), (8), (10) to (15), and (18).

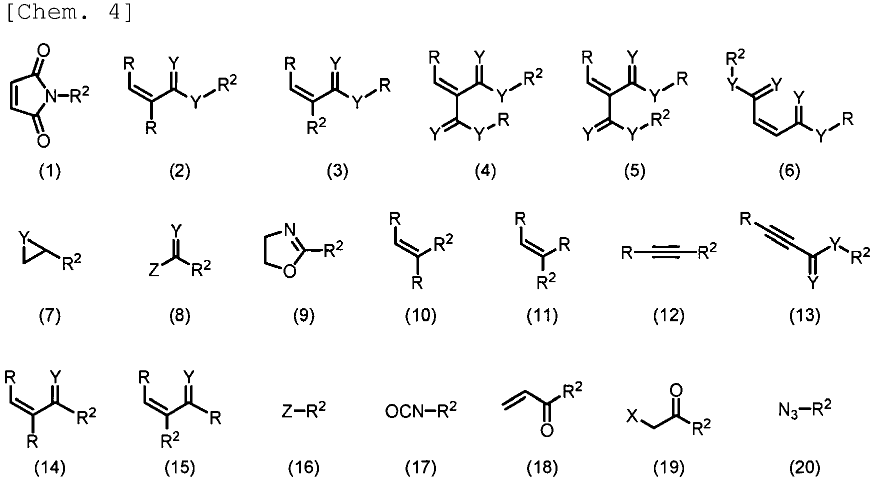

In formulas (1) to (20), R² represents the partial structure capable of plasticizing a protein. In formulas (2) to (8) and (13) to (15), Ys each independently represent an oxygen atom, a sulfur atom, or NR¹, and R¹ represents a hydrogen atom, a hydrocarbon group (for example, a C₁₋₆ alkyl group), an aromatic group (for example, a phenyl group, a naphthyl group, or a pyridyl group.), a carbonyl group, or a sulfonyl group. In formulas (2) to (6) and (10) to (15), Rs each independently represent a hydrogen atom, a hydrocarbon group (for example, a linear or branched C₁₋₆ alkyl group), or an aromatic group (for example, a phenyl group, a naphthyl group, or a pyridyl group.). In formulas (8) and (16), Z represents a hydrogen atom, a hydrocarbon group (for example, a C₁₋₆ alkyl group), an aromatic group (for example, a phenyl group, a naphthyl group, or a pyridyl group), a halogen atom (for example, a fluorine atom, a chlorine atom, a bromine atom, or an iodine atom), a sulfonic acid ester group, or a fluorine-containing carboxylic acid ester group. In general formula (19), X represents a halogen atom. The aromatic group may have a substituent of a halogen atom, a cyano group, a nitro group, an alkyl group (for example, a C₁₋₆ alkyl group), a haloalkyl group (for example, a trifluoromethyl group), or the like.

The use amount of the molecule capable of plasticizing a protein can be 1.5 to 7 equivalents per nucleophilic functional group contained in the protein. The use amount of the molecule capable of plasticizing a protein may be preferably 1.5 to 6.5 equivalents, 1.5 to 6 equivalents, 1.5 to 5.5 equivalents, 1.5 to 5 equivalents, 1.5 to 4 equivalents, 2 to 7 equivalents, 2 to 6.5 equivalents, 2 to 6 equivalents, 2 to 5.5 equivalents, 2 to 5 equivalents, 2 to 4.5 equivalents, 2.5 to 7 equivalents, 2.5 to 6.5 equivalents, 2.5 to 6 equivalents, 2.5 to 5.5 equivalents, 2.5 to 5 equivalents, or 2.5 to 4.5 equivalents per nucleophilic functional group. The "equivalent per nucleophilic functional group" means a molar equivalent of the molecule capable of plasticizing a protein to one nucleophilic functional group contained in the protein. By setting the use amount of the molecule capable of plasticizing a protein to a value within the above range, reactivity or reaction efficiency between the protein and the molecule capable of plasticizing a protein in the mechanochemical treatment is enhanced, and a target block copolymer is more efficiently obtained.

As described above, in a case of producing a prepolymer, the number of reactive functional groups (for example, electrophilic functional groups) of the molecule capable of plasticizing a protein contained in the mixture is made excessive with respect to the number of reactive functional groups (for example, nucleophilic functional groups) contained in the protein, whereby an unreacted reactive functional group remains in the molecule capable of plasticizing a protein after a binding reaction between the molecule capable of plasticizing a protein and the protein. This can also be achieved, for example, by adjusting the use amount (number of moles) of each of the protein and the molecule capable of plasticizing a protein contained in the mixture. That is, for example, the use amount (number of moles) of the molecule capable of plasticizing a protein contained in the mixture is made larger than the use amount (number of moles) of the protein. When the amount (number of moles) of the molecule capable of plasticizing a protein is large, the electrophilic functional group is excessive with respect to the nucleophilic functional group of the protein, and an unreacted reactive functional group can remain in the molecule capable of plasticizing a protein bound to the protein. Note that, when the number of reactive functional groups (for example, electrophilic functional groups) in the molecule capable of plasticizing a protein contained in the mixture is larger than the number of reactive functional groups (for example, nucleophilic functional groups) in the protein contained in the mixture, even if the used amount (number of moles) of the molecule capable of plasticizing a protein is the same as or smaller than the used amount (number of moles) of the protein, an unreacted reactive functional group can remain in the molecule capable of plasticizing a protein bound to the protein. This also makes it possible to produce a prepolymer.

The mechanochemical treatment is a treatment of performing a chemical reaction caused by directly absorbing mechanical energy. The mechanical energy may be exerted by, for example, an impact force or a shear force. Specifically, such a mechanochemical treatment is performed using a rolling ball mill, a medium stirring mill, a planetary mill, a jet mill, a mixer mill, an extruder (twin-screw extruder), or the like. Specifically, the protein and the molecule capable of plasticizing a protein are put in a grinding jar, and a medium ball or the like is put therein depending on the type of mill to be used. Thereafter, the grinding jar is set in a mixer mill apparatus and vibrated at a predetermined frequency and for a predetermined reaction time. At this time, a solvent, a base, a reaction accelerator, and the like may be further added to the grinding jar. Alternatively, the protein and the molecule capable of plasticizing a protein are put in a mixer mill and a milling procedure is initiated and the reactants are kneaded continuously. At this time, a solvent, a base, a reaction accelerator, and the like may be further added to the mixer mill. Alternatively, the protein and the molecule capable of plasticizing a protein are put in an extruder and kneaded. At this time, a solvent, a base, a reaction accelerator, and the like may be further added to the extruder. Note that, when the mechanochemical treatment is performed using the extruder, a target block copolymer can be continuously produced.

The frequency can be appropriately adjusted by a person skilled in the art depending on reaction, and may be, for example, 10 to 50 Hz, 10 to 45 Hz, 10 to 40 Hz, 10 to 35 Hz, 15 to 50 Hz, 15 to 45 Hz, 15 to 40 Hz, 15 to 35 Hz, 20 to 50 Hz, 20 to 45 Hz, 20 to 40 Hz, or 20 to 35 Hz.

A reaction time can be up to a time point at which the protein serving as a raw material disappears or detection of a block copolymer is observed at a certain level or more in an infrared (IR) absorption spectrum, gel filtration chromatography (GPC), or the like. The reaction time may be any time, and may be, for example, 30 to 240 minutes, 30 to 210 minutes, 30 to 180 minutes, 30 to 150 minutes, 30 to 120 minutes, 30 to 110 minutes, 30 to 100 minutes, 30 to 95 minutes, 60 to 240 minutes, 60 to 210 minutes, 60 to 180 minutes, 60 to 150 minutes, 60 to 120 minutes, 60 to 110 minutes, 60 to 100 minutes, 60 to 95 minutes, 70 to 240 minutes, 70 to 210 minutes, 70 to 180 minutes, 70 to 150 minutes, 70 to 120 minutes, 70 to 110 minutes, 70 to 100 minutes, 70 to 95 minutes, 80 to 240 minutes, 80 to 210 minutes, 80 to 180 minutes, 80 to 150 minutes, 80 to 120 minutes, 80 to 110 minutes, 80 to 100 minutes, or 80 to 95 minutes.

The solvent only needs to be a solvent capable of swelling the protein or a solvent capable of dissolving at least one of the protein and the molecule capable of plasticizing a protein, but the solvent should be a compound that is liquid at normal temperature and normal pressure and does not chemically react with the protein. A mechanochemical treatment to which such a solvent is added is known as liquid assisted grinding (LAG), and is a method for allowing a desired reaction to proceed more efficiently by adding a small amount of solvent into a grinding jar. Examples of the solvent include an alcohol-based solvent such as methanol or ethanol, and an aprotic polar solvent such as dimethyl sulfoxide (DMSO), N,N-dimethylformamide (DMF), N,N-dimethylacetamide (DMAc), N-methylpiperidone (NMP), or dihydrolevoglucosenone. The use amount of the solvent only needs to be, for example, 0.01 g to 1 g, and may be 0.01 g to 0.8 g, 0.01 g to 0.6 g, 0.01 g to 0.4 g, or 0.01 g to 0.2 g with respect to 1 g of the mass of the protein. In other words, the use amount of the solvent only needs to be, for example, 1 to 100 wt%, and may be 1 to 80 wt%, 1 to 60 wt%, 1 to 40 wt%, or 1 to 20 wt% with respect to the protein. It is considered that an extremely small amount of the solvent forms a microscopic reaction field by swelling the protein, locally bringing the protein into a dissolved state, or the like. Note that addition of the solvent is not necessarily required, and for example, when the molecule capable of plasticizing a protein is a liquid at normal temperature and normal pressure and has the above function as the solvent, the addition of the solvent may be unnecessary.

After completion of the reaction, the mixture is removed from the grinding jar, and may be washed with a solvent. By washing, an unreacted substance, excess byproducts generated by the reaction, and the like can be removed. Examples of a solvent used for washing include water, methanol, ethanol, acetonitrile, acetone, tetrahydrofuran, ethyl acetate, and hexane. After washing, the solvent used for washing may be distilled off by drying a product. Drying may be performed under reduced pressure.

An average particle size of the block copolymer according to the present embodiment is preferably 1 to 80 µm, 1 to 50 µm, 2 to 50 µm, 2 to 40 µm, 4 to 25 µm, 5 to 25 µm, 8 to 25 µm, or 8 to 16 µm. When the average particle size is in the above range, handleability is excellent. The average particle size is determined, for example, by the following method. Particles or powder of the block copolymer is uniformly dispersed on a glass plate by suction with a vacuum chamber, and then measured five times using a particle size analyzer for analysis of both a dry image and a wet image (trade name: DW-200nano, manufactured by JASCO International Corporation), a projection image is photographed with a 10 million pixel camera, and the obtained projection image is analyzed using image analysis software.

The block copolymer according to the present embodiment is obtained as a powder having a smaller average particle size and containing more uniform particles by mechanochemically treating a mixture containing a protein and a molecule capable of plasticizing a protein and then freeze-drying the mixture. An average particle size of a block copolymer powder obtained by freeze drying is, for example, about 1 to 30 µm, 1 to 20 µm, 1 to 10 µm, or 2 to 8 µm. Furthermore, such a powdery block copolymer has excellent handleability, and the use amount thereof can be finely adjusted. In addition, the freeze-dried block copolymer not only has a small average particle size but also is expected to disentangle the tertiary structure of the protein, and therefore, can be expected to improve dispersibility in an aqueous medium (aqueous liquid) such as water, a basic aqueous solution, an acidic aqueous solution, or a neutral aqueous solution containing an inorganic salt, and solubility in a solvent. In particular, when an aqueous dispersion obtained by dispersing a block copolymer powder obtained by a mechanochemical treatment or a block copolymer powder obtained by further freeze-drying the block copolymer powder in an aqueous medium is applied onto a surface of a base material, the aqueous dispersion can be more uniformly applied without unevenness. As a result, a more uniform coating film having as little unevenness as possible can be formed. That is, the aqueous dispersion of the block copolymer powder can also be useful as a coating liquid for forming a coating film. In addition, by applying an aqueous dispersion of the block copolymer powder onto a surface of a base material, bonding another base material thereto, and then curing the block copolymer, the two base materials can also be bonded to each other. In other words, the aqueous dispersion of the block copolymer according to the present embodiment can also be useful as a water-dispersible adhesive. Note that, when the aqueous dispersion of the block copolymer is used as a coating liquid, for example, a predetermined colorant or an additive generally used for a coating liquid or the like may be added to, blended with, or mixed with the aqueous dispersion of the block copolymer. In addition, when the aqueous dispersion of the block copolymer is used as a water-dispersible adhesive, an additive generally used for an adhesive or the like may be added to, blended with, or mixed with the aqueous dispersion of the block copolymer. The coating liquid or the water-dispersible adhesive containing the aqueous dispersion of the block copolymer according to the present embodiment uses an aqueous medium such as water as a solvent, and is therefore suitable for application to a material having low organic solvent resistance and adhesion of a material having low organic solvent resistance, and can reduce odor peculiar to an organic solvent.

In a block copolymer produced by a mechanochemical method, an organic solvent is not used in a production process thereof, or only an extremely small amount (an amount sufficient to swell a protein or a molecule capable of plasticizing a protein) of an organic solvent is used, and therefore the organic solvent is less likely to remain, and the block copolymer is easily purified as a powder. The block copolymer according to the present embodiment can also be purified as smaller particles having a uniform particle size distribution as described above.

When the block copolymer according to the present embodiment is applied to a piece of wood and the piece of wood is bonded to another piece of wood, an average elongation value in a tensile test is preferably 3.5% or more, 3.6% or more, 3.7% or more, 3.8% or more, 3.9% or more, 4.0% or more, 4.1% or more, 4.2% or more, 4.3% or more, 4.4% or more, 4.5% or more, 4.6% or more, or 4.7% or more. When the elongation value in the tensile test is 3.5% or more, it is considered that sufficient adhesiveness is exhibited. Since an organic solvent is less likely to remain in the block copolymer according to the present embodiment, the block copolymer according to the present embodiment is also suitable for adhesion of a material having low organic solvent resistance as compared with a conventional aqueous dispersion, water-dispersible adhesive, and coating liquid, and can reduce odor peculiar to an organic solvent.

Note that, when a block copolymer is produced by a conventional solution method, an organic solvent is easily incorporated into the block copolymer, and therefore it is difficult to remove the organic solvent even when the block copolymer is dried under reduced pressure, and the block copolymer is formed into a large lump. Hardness of such a block copolymer is lower than that of a protein alone due to an influence of imparting a partial structure having plasticity to the molecule capable of plasticizing a protein, and therefore the block copolymer cannot be pulverized into a powder having a uniform particle size distribution due to an influence of difficulty in pulverization, and it is difficult to apply the block copolymer to a surface of a base material at a uniform thickness. In addition, since the block copolymer cannot be sufficiently dried even by freeze-drying or the like, it is difficult to obtain the block copolymer as a powder having a small particle size and exhibiting a uniform particle size distribution, and it is also difficult to maintain equivalent quality and to produce the block copolymer with favorable reproducibility.

### [Prepolymer and finished polymer]

As described above, by the production method according to the present embodiment, a block copolymer can be produced as a finished polymer or a prepolymer.

The prepolymer is a polymer having an unreacted reactive functional group in which a polymerization reaction is stopped at an appropriate intermediate stage in order to facilitate molding, and is generally produced by stopping the polymerization reaction in a state having plasticity suitable for an application. Such a prepolymer can be adjusted to have a desired shape by allowing the remaining polymerization reaction to proceed while being molded. The prepolymer of the block copolymer has an unreacted reactive functional group (for example, an electrophilic functional group), and any structure can be easily formed between the block copolymers by causing the unreacted reactive functional group to react with, for example, another compound having a nucleophilic reactive functional group. In addition, in the prepolymer of the block copolymer, by binding a functional group capable of exerting a desired function to an unreacted reactive functional group, a desired function according to a functional group can be exerted in a finished block copolymer. In addition, the prepolymer of the block copolymer can be expected to have improved solubility in a predetermined solvent.

The prepolymer of the block copolymer having two or more unreacted electrophilic functional groups may be further mixed with a compound having two or more nucleophilic functional groups to produce a finished polymer. By reaction of the prepolymer having two or more unreacted electrophilic functional groups with a compound having two or more nucleophilic functional groups, a plurality of prepolymers are linked to each other to form a block copolymer (finished polymer) having a higher degree of polymerization.

The compound having two or more nucleophilic functional groups only needs to be a substance having two or more nucleophilic functional groups in its chemical structure, and may be selected from molecules exemplified as the above-described molecule capable of plasticizing a protein. Examples of the nucleophilic functional group include a hydroxy group, an amino group, and a thiol group.

Examples of the compound having two or more nucleophilic functional groups include a diol such as polyethylene glycol, polypropylene glycol, or polybutylene glycol, a dithiol such as dithiothreitol or 2,2'-(ethylenedioxy) diethanethiol, and a diamine such as polyethylene diamine.

The use amount of the compound having two or more nucleophilic functional groups can be 1.5 to 7 equivalents per nucleophilic functional group contained in the prepolymer. The use amount of the compound having two or more nucleophilic functional groups may be preferably 1.5 to 6.5 equivalents, 1.5 to 6 equivalents, 1.5 to 5.5 equivalents, 1.5 to 5 equivalents, 1.5 to 4 equivalents, 2 to 7 equivalents, 2 to 6.5 equivalents, 2 to 6 equivalents, 2 to 5.5 equivalents, 2 to 5 equivalents, 2 to 4.5 equivalents, 2.5 to 7 equivalents, 2.5 to 6.5 equivalents, 2.5 to 6 equivalents, 2.5 to 5.5 equivalents, 2.5 to 5 equivalents, or 2.5 to 4.5 equivalents per nucleophilic functional group.

The block copolymer (finished polymer) can be produced, for example, by dissolving a prepolymer in a solvent (for example, DMSO), adding a compound having two or more nucleophilic functional groups thereto, and stirring the mixture. When a progress of a polymerization reaction is slow, the reaction solution may be heated. As one aspect of a method for producing a finished polymer, a resin film can be produced by dissolving a prepolymer in a solvent, adding a compound having two or more nucleophilic functional groups thereto, then casting the mixture onto a surface of a base material, and heating and drying the casted solution.

One embodiment of the present invention is a method for producing a molded body, the method including: a step of mechanochemically treating a mixture containing a protein and a molecule having a function of plasticizing a protein to obtain a block copolymer; and a step of molding the block copolymer. In the method for producing a molded body according to the present embodiment, before molding the block copolymer, a composition containing the block copolymer may be obtained, and then the composition may be molded.

The block copolymer according to the present embodiment can be formed, for example, as a solid composition alone. In addition, the block copolymer can be formed as a solid or liquid composition by blending, adding, or mixing another solid or liquid component. The other component contained in the composition of the block copolymer is not particularly limited, and any known component can be adopted. Note that the solid composition of the block copolymer may be in a form of, for example, a powder, a particle, a lump, or a gel. The liquid composition of the block copolymer may be in a form of, for example, a solution or a dispersion. As the other component contained in the liquid composition of the block copolymer, water, an organic solvent, an ionic liquid, and a supercritical liquid liquid, which are generally used when a solid is formed as a liquid composition, can be adopted.

The composition of the block copolymer can be used as a molding material for obtaining a predetermined molded body. Examples of the molded body molded using the composition of the block copolymer as a molding material include a fiber, a film, a resin, a gel, a porous body, and a particle. These molded bodies can be molded, for example, according to a known molding method using a composition containing a protein material as a molding material. The fiber is obtained by a known spinning method such as a wet spinning method, a dry-wet spinning method, or a dry spinning method using a dope solution containing a composition of a block copolymer (preferably, a powdery composition composed only of a block copolymer) and a solvent. The resin can be molded (produced) by heating and pressurizing a composition of a block copolymer (preferably, a powdery composition composed only of a block copolymer) according to a method described in, for example, JP 6830604 B2. The film can be molded (produced) according to a method described in, for example, JP 5678283 B2. Furthermore, the gel, the porous body, the particle, and the like can be molded (produced) according to methods described in, for example, JP 5782580 B2, JP 5796147 B2, and JP 5823079 B2. Note that the resin can also be obtained, for example, by removing, from a composition (for example, gel-like) containing a block copolymer and a predetermined solvent or dispersion medium, the solvent or the dispersion medium to solidify the composition.

The block copolymer according to the present embodiment can be used as an adhesive for bonding an adherend or a coating agent for laminating a coating layer on a base material in a solution state, an aqueous dispersion state, a film state, or a powder state containing the block copolymer as a main component. The solution-state adhesive or the solution-state coating agent, the water-dispersible adhesive or the water-dispersible coating agent, the film-shaped adhesive or the film-shaped coating agent, the powdery adhesive or the powdery coating agent can be obtained by, for example, the following production method.

The solution-state adhesive or the solution-state coating agent containing the block copolymer can be produced by a method including a step of dissolving a block copolymer obtained by the present embodiment method in a solvent. According to such a method, unlike a conventional solution-state adhesive or solution-state coating agent containing a petroleum-derived component, a biodegradable solution-state adhesive or solution-state coating agent can be easily obtained.

Examples of a solvent used in production of the solution-state adhesive or the solution-state coating agent include an aqueous medium such as water in which a block copolymer can be dissolved, a basic aqueous solution, an acidic aqueous solution, or a neutral aqueous solution containing an inorganic salt, and an organic solvent. Examples of the organic solvent include formic acid, dimethyl sulfoxide (DMSO), hexafluoroisopropanol (HFIP), N,N-dimethylformamide (DMF), N,N-dimethylacetamide (DMAc), N-methylpiperidone (NMP), and dihydrolevoglucosenone.

The block copolymer contained in the solution-state adhesive or the solution-state coating agent is obtained by the production method according to the present embodiment, and is not particularly limited as long as the block copolymer can be dissolved in the aqueous medium or organic solvent described above. An adhesive or a coating agent in which the block copolymer is dissolved in an aqueous medium has an advantage that handling properties and the like are superior to those of an adhesive or a coating agent in which the block copolymer is dissolved in an organic solvent. Note that, in the block copolymer according to the present embodiment, since the molecule capable of plasticizing a protein binds to the protein, the polymer compound exhibits higher water solubility than the protein. Therefore, an aqueous adhesive or an aqueous coating agent in which the block copolymer is dissolved in an aqueous medium can have a higher protein content than an aqueous adhesive or an aqueous coating agent in which a protein is simply dissolved in an aqueous medium. Therefore, when an aqueous adhesive or an aqueous coating agent in which the block copolymer is dissolved in an aqueous medium is used, it can be expected that adhesive strength of the adhesive or the coating agent to an adhesion surface of an adherend or a laminate surface described later is improved as compared with a case of using an aqueous adhesive or an aqueous coating agent in which a protein is dissolved in an aqueous medium.

A concentration of the block copolymer in a solution-state adhesive or a solution-state coating agent is appropriately determined based on a solubility of the block copolymer in a solvent or the like. The concentration may be, for example, 5 to 40% by mass, 10 to 35% by mass, 15 to 20% by mass, or 20 to 25% by mass. In addition, the solution-state adhesive or the solution-state coating agent may contain, as necessary, a component other than the block copolymer, such as various additives contained in a known solution-state adhesive or solution-state coating agent.

The solution-state adhesive containing the block copolymer is used for producing an adhesive body in which a plurality of adherends is bonded to each other. For example, the solution-state adhesive is interposed between a plurality of adherends to be bonded, then a solvent in the solution-state adhesive is removed to solidify the block copolymer, whereby the adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that a material of the adherend is not particularly limited as long as the adherends can be bonded to each other with a solution-state adhesive containing a block copolymer, and the adherend may be an organic substance (for example, a cellulose product such as paper or wood, a synthetic resin, or a protein product) or an inorganic substance (a metal or a non-metal such as glass).

A specific method for producing the adhesive body using the solution-state adhesive is not limited at all. For example, the solution-state adhesive is put on an adhesion surface by applying or dropping the solution-state adhesive to an adhesion surface of at least one of adherends to be bonded to each other, or by bringing the adhesion surfaces of the adherends into contact with a liquid surface of the solution-state adhesive or immersing the adhesion surfaces in the liquid surface, then the adherends are superimposed on each other or caused to butt against each other at the adhesion surfaces, whereby the solution-state adhesive may be interposed between the plurality of adherends. In addition, when the solvent in the solution-state adhesive interposed between the plurality of adherends is removed to solidify the block copolymer, for example, heating, air drying, or natural drying may be performed in a state where pressure is applied to the plurality of adherends from at least one of a superimposing direction and a butting direction of the adhesion surfaces such that the superimposed or butted adhesion surfaces are brought into close contact with each other. Of course, when bonding is possible without applying such pressure, it is not necessary to apply pressure.

The solution-state coating agent containing the block copolymer is used for producing a laminate in which a coating layer is laminated on the whole or a part of a surface of a base material. For example, the solution-state coating agent is supplied to at least a part of a surface of a base material on which a coating layer is to be laminated, the surface part of the base material is coated with the solution-state coating agent, and then a solvent in the solution-state adhesive is removed to solidify the block copolymer. This makes it possible to obtain a laminate by laminating the coating layer on at least a part of the surface of the base material. Also by such a method, a laminate can be obtained using a biodegradable coating agent, and therefore there is an advantage that a laminate capable of reducing an environmental load at the time of disposal can be easily produced. Note that a material of the base material is not particularly limited as long as a solution-state coating agent containing the block copolymer can be bonded to the base material, and the base material may be similar to an adherend to be bonded with the solution-state adhesive described above.

A specific method for producing a laminate using the solution-state coating agent is also not limited at all. For example, by applying or dropping the solution-state coating agent to at least a part of a surface of the base material, bringing at least a part of the surface of the base material into contact with a liquid surface of the solution-state coating agent, or immersing at least a part of the surface of the base material in the liquid surface, the solution-state coating agent may be supplied to at least a part of the base material so as to form a layer having a predetermined thickness to perform coating. When the block copolymer in the coating agent layer (solution-state coating agent) laminated on the surface of the base material is solidified, for example, the coating agent layer on the surface of the base material may be heated, air-dried, or naturally dried to remove the solvent in the coating agent layer. Note that, at this time, a predetermined pressing body (pressurizing body) may be placed on the coating agent layer so as to cover the entire surface of the coating agent layer, as necessary, and the coating agent layer may be pressed (pressurized) against the surface of the base material such that the coating agent layer is in close contact with the surface of the base material. At this time, it is preferable to prevent the pressing body from being bonded to the solution-state coating agent. For example, by applying a release agent to a contact surface of the pressing body with the coating agent layer, attaching release paper to the contact surface, performing a surface treatment such that the coating agent is not bonded to the contact surface, or using a material that is not bonded to the solution-state coating agent as the pressing body, the adhesion of the solution-state coating agent to the pressing body can be prevented.

The water-dispersible adhesive or the water-dispersible coating agent containing the block copolymer can be produced by a method including a step of dispersing a block copolymer obtained by the present embodiment method in an aqueous medium. According to such a method, unlike a conventional water-dispersible adhesive or water-dispersible coating agent containing a petroleum-derived component, a biodegradable water-dispersible adhesive or water-dispersible coating agent can be easily obtained.

Examples of the aqueous medium used for producing the water-dispersible adhesive or the water-dispersible coating agent include water in which the block copolymer can be dispersed, a basic aqueous solution, an acidic aqueous solution, and an aqueous solution containing an inorganic salt.

The block copolymer contained in the water-dispersible adhesive or the water-dispersible coating agent is obtained by the production method according to the present embodiment, and is not particularly limited as long as the block copolymer can be dispersed in the aqueous medium described above. The content of the block copolymer in the water-dispersible adhesive or the water-dispersible coating agent is appropriately determined in consideration of adhesiveness of the water-dispersible adhesive to an adherend, adhesiveness or fixability of the water-dispersible coating agent to a base material, and the like. In addition, the water-dispersible adhesive and the water-dispersible coating agent may contain, as necessary, a component other than the block copolymer, such as various additives contained in a known water-dispersible adhesive or water-dispersible coating agent.

Note that, in the block copolymer according to the present embodiment, since the molecule capable of plasticizing a protein binds to the protein, the block copolymer exhibits higher affinity for an aqueous medium containing water than the protein. Therefore, in such a water-dispersible adhesive or water-dispersible coating agent containing the block copolymer, the content (dispersion amount) of a protein can be increased and the block copolymer can be uniformly dispersed in an aqueous medium as compared with a water-dispersible adhesive or a water-dispersible coating agent in which a protein is simply dispersed in an aqueous medium. Therefore, when a water-dispersible adhesive or a water-dispersible coating agent in which the block copolymer is dispersed in an aqueous medium is used, it can be expected that adhesive strength of the adhesive or the coating agent to an adhesion surface of an adherend or a laminate surface described later is improved as compared with a case of using a water-dispersible adhesive or a water-dispersible coating agent in which a protein is dispersed in an aqueous medium.

The water-dispersible adhesive containing the block copolymer is used for producing an adhesive body in which a plurality of adherends is bonded to each other. For example, the water-dispersible adhesive is interposed between a plurality of adherends to be bonded, then an aqueous medium in the water-dispersible adhesive is removed to solidify the block copolymer, whereby adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that a material of the adherend is not particularly limited as long as the adherend can be bonded with the water-dispersible adhesive containing the block copolymer, and the adherend may be similar to an adherend to be bonded with the solution-state adhesive containing the block copolymer.

A specific method for producing the adhesive body using the water-dispersible adhesive is not also limited at all. For example, when the water-dispersible adhesive is interposed between a plurality of adherends to be bonded, a similar method to the method used when the solution-state adhesive is interposed between a plurality of adherends can be adopted. In addition, also when the aqueous medium in the water-dispersible adhesive interposed between the adherends is removed to solidify the block copolymer, a similar method to the method used when the solvent in the solution-state adhesive interposed between the adherends is removed to solidify the block copolymer can be adopted.

The water-dispersible coating agent containing the block copolymer is used for producing a laminate in which a coating layer is laminated on the whole or a part of a surface of a base material. For example, the water-dispersible coating agent is supplied to at least a part of a surface of a base material on which a coating layer is to be laminated, the surface part of the base material is coated with the solution-state coating agent, and then the aqueous medium in the water-dispersible adhesive is removed to solidify the block copolymer. This makes it possible to obtain a laminate by laminating the coating layer on at least a part of the surface of the base material. Also by such a method, a laminate can be obtained using a biodegradable coating agent, and therefore there is an advantage that a laminate capable of reducing an environmental load at the time of disposal can be easily produced. Note that a material of the base material is not particularly limited as long as the water-dispersible coating agent containing the block copolymer can be bonded to the base material, and the base material may be similar to a base material on which a coating layer is formed with the solution-state coating agent described above.

A specific method for producing a laminate using the water-dispersible coating agent is not also limited at all. For example, when the water-dispersible coating agent is supplied to at least a part of the surface of the base material, a similar method to the method used when the above-described solution-state coating agent is supplied to the surface of the base material can be adopted. In addition, when the aqueous medium in the water-dispersible coating agent supplied to the surface of the base material is removed to solidify the block copolymer, a method similar to the method used when the solvent in the solution-state coating agent supplied to the surface of the base material is removed to solidify the block copolymer can be adopted.

The film-shaped adhesive or the film-shaped coating agent containing the block copolymer can be produced by a method including a step of molding a film from a block copolymer solution in which a block copolymer obtained by the present embodiment method is dissolved. According to such a method, unlike a conventional film-shaped adhesive or film-shaped coating agent containing a petroleum-derived component, a biodegradable film-shaped adhesive or film-shaped coating agent having can be easily obtained. Note that a method for molding the film may be cast molding.

As a film forming solution used for producing the film-shaped adhesive or the film-shaped coating agent, for example, a block copolymer solution used as the solution-state adhesive or the solution-state coating agent is suitably used. When the block copolymer solution is cast-molded, a known method and a known condition similar to those when a protein solution is cast-molded can be adopted.

The film-shaped adhesive containing the block copolymer is used for producing an adhesive body in which a plurality of adherends is bonded to each other. For example, in a state where a film-shaped adhesive is interposed between a plurality of adherends to be bonded, the film-shaped adhesive is swollen or heated to be softened, and then the film-shaped adhesive is cured in a state of being brought into pressure contact with the adherends, whereby the adherends can be bonded to each other to obtain an adhesive body. In addition, the film-shaped adhesive may be swollen or heated to be softened in advance, interposed between a plurality of adherends, and then cured in a state of being brought into pressure contact with the adherends. According to these methods, since an adhesive body is obtained using a biodegradable adhesive, there is an advantage that an adhesive body capable of reducing an environmental load at the time of disposal can be easily produced. Note that the adherend may be any adherend as long as the adherend can be bonded with the film-shaped adhesive containing the block copolymer, and the adherend may be similar to an adherend to be bonded with the solution-state adhesive containing the block copolymer.

The film-shaped adhesive is softened when it absorbs moisture or is heated, and is cured by subsequent drying or cooling. In addition, when the block copolymer contained in the film-shaped adhesive has a property of exhibiting a shrinkage behavior by contact with water or heating particularly when being formed into a molded body, the film-shaped adhesive also shrinks by contact with water or heating. Therefore, when the film-shaped adhesive containing the block copolymer is interposed between adherends in a state of being swollen or heated to be softened, the film-shaped adhesive partially bites into a gap present on an adhesion surface of each of the adherends, and in particular, when the modified protein has the above-described shrinkage property, the film-shaped adhesive shrinks in a state of biting into the gap of the adhesion surface. When the film-shaped adhesive is cured in this state, it is considered that the film-shaped adhesive and the adherends are bonded to each other by an anchor effect, and the adherends are bonded to each other. In addition, the film-shaped adhesive exhibits sufficient flexibility by containing, as a main component, a block copolymer in which the molecule that plasticizes a protein is bound to the protein. Therefore, such a film-shaped adhesive exhibits higher flexibility when being softened, and thus more sufficiently bites into the gap of the adhesion surface of each of the adherends, and as a result, it can be expected that higher adhesive strength can be obtained. Moreover, since the film-shaped adhesive has excellent flexibility, the film-shaped adhesive can be peeled off from the adherend after being bonded to the adherend, and further exhibits an excellent function of being reusable as the film-shaped adhesive. Note that when the film-shaped adhesive is reused, the film-shaped adhesive may be washed with water after being peeled off from the adherend. Note that it is also considered that adhesion of the film-shaped adhesive to the adherend is caused by hydrogen bonding between the adherend and the block copolymer.

When the film-shaped adhesive is swollen to be softened, an aqueous liquid such as water that is absorbed by the film-shaped adhesive and can shrink the film-shaped adhesive, or a solution or a dispersion containing water is suitably used. Then, for example, by causing the film-shaped adhesive to absorb water by dropping the above-described aqueous liquid or immersing a plurality of adherends in the aqueous liquid, the film-shaped adhesive may be swollen to be softened. In addition, when the film-shaped adhesive softened by swelling is cured, for example, a method for heating and drying, air-drying, or naturally drying the film-shaped adhesive in a state where the film-shaped adhesive is interposed between adherends can be adopted.

When the film-shaped adhesive is heated to be softened, the film-shaped adhesive may be heated before the film-shaped adhesive is interposed between adherends, or the whole of the adherends and the film-shaped adhesive may be heated after the film-shaped adhesive is interposed between the adherends. A heating temperature is not particularly limited as long as the heating temperature is a temperature at which the film-shaped adhesive can be softened and a protein contained in the film-shaped adhesive is not adversely affected (for example, not decomposed). When the film-shaped adhesive softened by heating is cured, a method for cooling the film-shaped adhesive with a cooling device or allowing the film-shaped adhesive to cool can be adopted.

In order to cause the film-shaped adhesive softened by swelling or heating to sufficiently bite into a gap of an adhesion surface of the adherend, it is desirable to bring an interface between the softened film-shaped adhesive and the adherend into close contact with each other. Therefore, for example, it is preferable to apply pressure to a plurality of adherends from at least one of an overlapping direction and a butting direction of the adhesion surfaces to cure the softened film-shaped adhesive while pressing the softened film-shaped adhesive against the adherends. Any general method can be adopted as the method for pressurizing the adherends here.

The film-shaped coating agent containing the block copolymer is used for producing a laminate in which a coating layer is laminated on the whole or a part of a surface of a base material. For example, in a state where the film-shaped coating agent is placed so as to cover at least a part of a surface of the base material, the film-shaped coating agent is swollen or heated to be softened, and then cured in a state of being brought into pressure contact with the surface of the base material, whereby a coating layer can be laminated on at least a part of the surface of the base material to obtain a laminate. In addition, the film-shaped coating agent may be swollen or heated to be softened in advance, placed so as to cover so as to cover at least a part of the surface of the base material, and then cured in a state of being brought into pressure contact with adherends. According to these methods, since a laminate is obtained using a biodegradable coating agent, there is an advantage that a laminate capable of reducing an environmental load at the time of disposal can be easily produced. Note that the base material may be any material as long as the film-shaped coating agent containing the block copolymer can be bonded to the base material, and the base material may be similar to an adherend to be bonded with the film-shaped adhesive containing the block copolymer.

A mechanism by which the film-shaped coating agent is bonded to the surface of the base material is considered to be similar to a mechanism by which the film-shaped adhesive is bonded to an adhesion surface of the adherend. Therefore, as a specific method for producing a laminate using the film-shaped coating agent, a similar method to the method used when an adhesive body is obtained using the film-shaped adhesive can be adopted. Note that, when a softened film-shaped coating agent is cured while being brought into pressure contact with the surface of the base material, for example, the film-shaped coating agent may be pressed against the surface of the base material using a pressing body similar to a pressing body used when the solution-state coating agent with which a top surface of the base material is coated is pressed against the surface of the base material. Similarly to the film-shaped adhesive, the film-shaped coating agent not only can be expected to have high adhesive strength to a base material, but also can be peeled off after being bonded, and furthermore can be reused.

As the powdery adhesive or the powdery coating agent containing a block copolymer, a powder composition containing a powder of a block copolymer obtained by the present embodiment method is used. This powder composition may contain auxiliary components such as various addition residues as long as the powder composition contains the block copolymer as a main component.

The block copolymer contained in the powdery adhesive or the powdery coating agent is not particularly limited as long as the block copolymer is obtained by the production method according to the present embodiment. For example, any of block copolymers contained in a solution-state adhesive or a solution-state coating agent, a water-dispersible adhesive or a water-dispersible coating agent, and a film-shaped adhesive or a film-shaped coating agent can be used. Note that, as described above, the block copolymer according to the present embodiment exhibits higher affinity for an aqueous medium (aqueous liquid) containing water than a protein. Therefore, in such a powder adhesive or powdery coating agent containing the block copolymer, the content (dispersion amount) of a protein can be increased and the block copolymer can be uniformly dispersed in an aqueous medium as compared with a water-dispersible adhesive or a water-dispersible coating agent in which a protein is simply dispersed in an aqueous medium. Therefore, when a water-dispersible adhesive or a water-dispersible coating agent in which the block copolymer is dispersed in an aqueous medium is used, it can be expected that adhesive strength of the adhesive or the coating agent to an adhesion surface of an adherend or a laminate surface described later is improved as compared with a case of using a water-dispersible adhesive or a water-dispersible coating agent in which a protein is dispersed in an aqueous medium.

The powdery adhesive containing the modified protein is also used for producing an adhesive body in which a plurality of adherends is bonded to each other. For example, in a state where the powdery adhesive is interposed between a plurality of adherends to be bonded, the powdery adhesive is heated and pressurized via the adherends to solidify the powdery adhesive, whereby the adherends can be bonded to each other to obtain an adhesive body. According to such a method, since an adhesive body is obtained using a biodegradable adhesive, there is an advantage that an adhesive body capable of reducing an environmental load at the time of disposal can be easily produced. Note that the adherend may be any adherend as long as the adherend can be bonded with the powdery adhesive containing the block copolymer, and for example, the adherend may be similar to an adherend to be bonded with the solution-state adhesive containing the block copolymer.

A specific method for producing the adhesive body using the powdery adhesive is not also limited at all. For example, when the powdery adhesive interposed between a plurality of adherends to be bonded is heated and pressurized, the adherends are sandwiched by metal plates and the like from both sides opposite to the adhesion surface side, and the metal plates are heated to heat the powdery adhesive interposed between the adherends together with the adherends. At the same time, the metal plates are pressurized with a hand press machine or the like, and the powdery adhesive is pressurized for a predetermined time via the adherends. As a result, the powdery adhesive is converted into a resin and solidified to bond the adherends. Note that a heating temperature, a pressurization amount, and a heating and pressurization time when the powdery adhesive is solidified are appropriately selected from a range in which the modified protein can be converted into a resin according to the type of modified protein contained in the powdery adhesive.

The powdery coating agent containing the block copolymer is also used for producing a laminate in which a coating layer is laminated on the whole or a part of a surface of a base material. For example, in a state where the powdery coating agent is placed on at least a part of a surface of a base material, the powdery coating agent is heated and pressurized between a pressurizing body and the base material to be solidified, whereby a coating layer is laminated on at least a part of the surface of the base material.

A specific method for producing a laminate using the powdery coating agent is not also limited at all. For example, a metal plate or the like is disposed so as to cover the entire powdery adhesive placed on the surface of the base material, and the metal plate is heated to heat the powdery adhesive. At the same time, the powdery adhesive is pressurized between the metal plate and the base material for a predetermined time with a hand press machine or the like, whereby the powdery coating agent is converted into a resin and solidified. Note that a heating temperature, a pressurization amount, and a heating and pressurization time of the powdery coating agent are similar to those when an adhesive body is obtained using the powdery adhesive.

### Examples

Hereinafter, the present disclosure will be described more specifically based on Examples and the like. Note that the present disclosure is not limited to the following Examples.

Abbreviations used in Examples are used in a sense commonly used in the field of organic chemistry or genetic engineering unless otherwise stated. Some examples are described below.
DCM: dichloromethane
DMSO: dimethyl sulfoxide
GPC: gel filtration chromatography
HFIP: hexafluoroisopropanol
PEG: polyethylene glycol
RO water: water treated using reverse osmosis membrane (RO membrane)
TCEP: tris(2-carboxyethyl) phosphine
THF: tetrahydrofuran

In Examples or Comparative Examples, a compound represented by the following formula (hereinafter, also referred to as "dimaleimide PEG") was used as a molecule capable of plasticizing a protein. In the formula, n is 454.

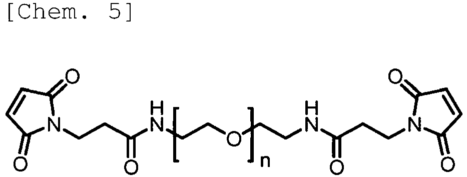

### 1. Protein block copolymer reaction

### (1) Preparation of block copolymer

As described later, a protein having two thiol groups and polyethylene glycol having two maleimide groups (dimaleimide PEG) were caused to react with each other by a solution method or a mechanochemical method to prepare a block copolymer. The obtained block copolymer was evaluated using GPC. The following formula is a schematic reaction diagram of a protein having two thiol groups and PEG having two maleimide groups.

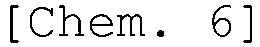

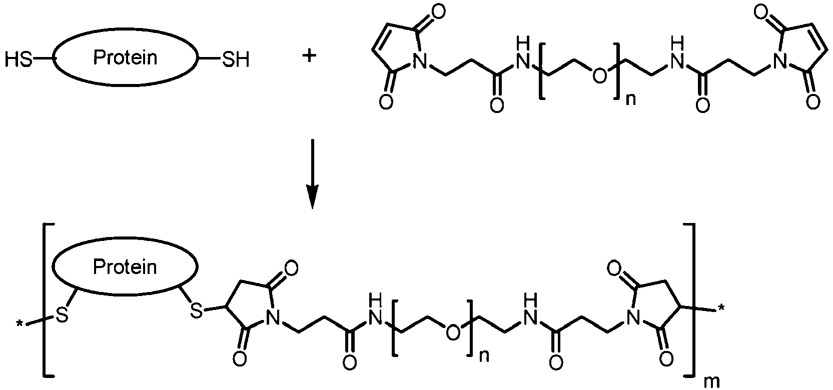

### Comparative Example 1: Preparation of block copolymer by solution method

Protein PRT2662 of 27461 Da (SEQ ID NO: 8, 255 mg, 9.3 µmol) and dimaleimide PEG of about 20 kDa (186 mg, 9.3 µmol) were suspended in DMSO (4.6 mL). Thereafter, while the mixture was heated to 100°C in an oil bath, the mixture was stirred for 15 minutes using a Hercules stirrer (trade name: MODEL HERAXLES/16G, manufactured by Koike Precision Instruments Co., Ltd.) to cause the protein to react with dimaleimide PEG, thereby obtaining a brown and transparent solution in which a reactant thereof was dissolved. Thereafter, the obtained solution was thinly applied to a metal plate to which a release film (manufactured by Teijin Film Solution Co., Ltd., thickness: 38 µm) was attached using a doctor blade (manufactured by Imoto machinery Co., Ltd., coating width: 80 mm, gap: 400 µm) to obtain a coating film. Subsequently, the coating film was dried at 60°C for two hours or more using a blown constant temperature thermostat, and furthermore, the solvent was removed at 80°C for 15 hours using a vacuum oven to obtain a brown and transparent film-shaped block copolymer (Comparative Example 1).

### Example 1: Preparation of block copolymer by mechanochemical method (addition of water)

Using a pulverizer (trade name: Mixer Mill MM400, manufactured by Verder Scientific Co., Ltd.), protein PRT2662 of 27461 Da (100 mg, 3.6 µmol) powder, and dimaleimide PEG of about 20 kDa (73 mg, 3.6 µmol) powder, the dimaleimide PEG powder was put in a 1.5 mL volume ball mill container of the pulverizer, and then RO water (100 µL) as a swelling solvent was dropped onto the powder in the container to wet the entire powder. Subsequently, ten 2 mmφ balls (manufactured by AS ONE Corporation) were put therein and the container was hermetically sealed, and then the pulverizer was vibrated at a frequency of 30 Hz to cause the protein to react with the dimaleimide PEG. After three hours, the reactants were vacuum-dried at room temperature to obtain a white powdery block copolymer (Example 1).

### Example 2: Preparation of block copolymer by mechanochemical method (addition of DMSO)

Protein PRT2662 of 27461 Da (100 mg, 3.6 µmol) and dimaleimide PEG of about 20 kDa (73 mg, 3.6 µmol) were put in the 1.5 mL volume ball mill container of the same grinder as used in preparing the block copolymer of Example 1, and then DMSO (100 µL) as a swelling solvent was dropped thereto to wet the entire powder. Ten 2 mmφ balls (manufactured by AS ONE Corporation) were put therein and the container was hermetically sealed, and then the pulverizer was vibrated at a frequency of 30 Hz to cause the protein to react with the dimaleimide PEG. After three hours, the reactants were vacuum-dried at room temperature to obtain a white powdery block copolymer (Example 2).

### Example 3: Preparation of block copolymer by mechanochemical method (addition of ethanol)

Protein PRT2662 of 27461 Da (100 mg, 3.6 µmol) and dimaleimide PEG of about 20 kDa (73 mg, 3.6 µmol) were put in the 1.5 mL volume ball mill container of the same grinder as used in preparing the block copolymer of Example 1, and then ethanol (100 µL) as a swelling solvent was dropped thereto to wet the entire powder. Ten 2 mmφ balls (manufactured by AS ONE Corporation) were put therein and the container was hermetically sealed, and then the pulverizer was vibrated at a frequency of 30 Hz to cause the protein to react with the dimaleimide PEG. After three hours, the reactants were vacuum-dried at room temperature to obtain a white powdery block copolymer (Example 3).

### Example 4: Preparation of block copolymer by mechanochemical method (addition of methanol)

Protein PRT2662 of 27461 Da (100 mg, 3.6 µmol) and dimaleimide PEG of about 20 kDa (73 mg, 3.6 µmol) were put in the 1.5 mL volume ball mill container of the same grinder as used in preparing the block copolymer of Example 1, and then methanol (100 µL) as a swelling solvent was dropped thereto to wet the entire powder. Ten 2 mmφ balls (manufactured by AS ONE Corporation) were put therein and the container was hermetically sealed, and then the pulverizer was vibrated at a frequency of 30 Hz to cause the protein to react with the dimaleimide PEG. After three hours, the reactants were vacuum-dried at room temperature to obtain a white powdery block copolymer (Example 4).

### (2) Evaluation

### (2-1) Appearances of block copolymer film and block copolymer powder

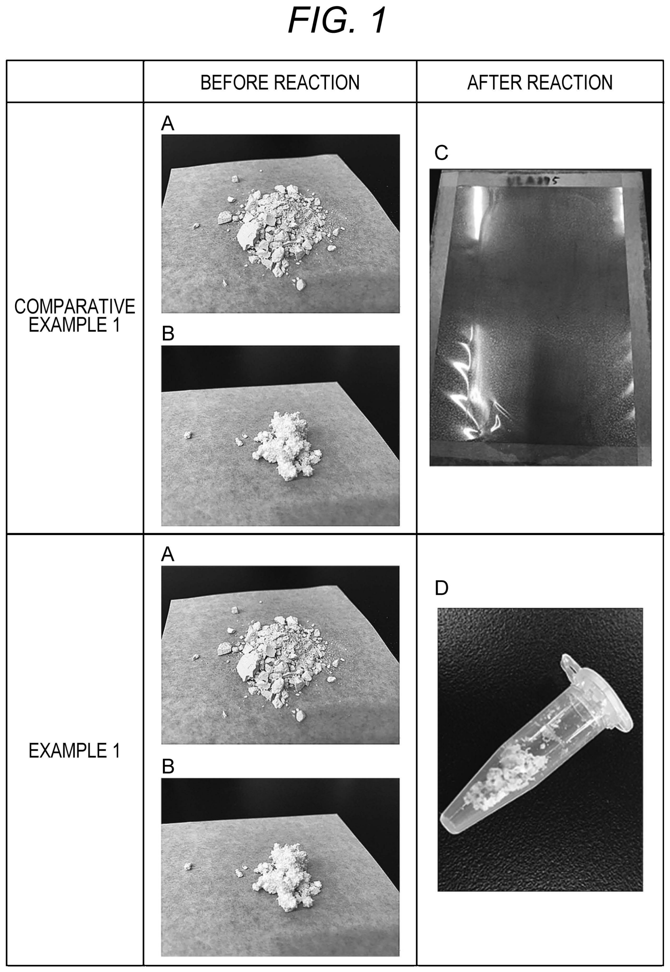

Fig. 1 is a photograph illustrating an appearance of the block copolymer film of Comparative Example 1 and an appearance of the block copolymer powder of Example 1. In Fig. 1, A is a photograph illustrating a powder of protein PRT2662 before reaction, B is a photograph illustrating a powder of dimaleimide PEG before reaction, C is a photograph illustrating the block copolymer film of Comparative Example 1, and D is a photograph illustrating an appearance of the block copolymer powder of Example 1.

### (2-2) GPC measurement

In GPC, an analyte was separated using an Agilent 1260 Infinity II liquid chromatography system (Agilent Technologies, Inc.) equipped with a refractive index (RI) detector, a styrene-divinylbenzene copolymer column (inner diameter: 4.6 mm × 150 mm) stationary phase equipped with a 0.5 µm guard column filter (trade name: Shodex GPC HK-G, manufactured by Showa Denko K.K.), and HFIP. A mobile phase was monitored using an Agilent 1260 Infinity II refractive index detector (RID) (Agilent Technologies, Inc.) at 40°C with a flow rate of 0.15 mL/min. A protein was analyzed as an HFIP solution to which 0.1 wt% sodium trifluoroacetate had been added (final concentration: 1 mM). To a protein having a cysteine residue, TCEP was added in order to prevent formation of a disulfide bond (final concentration: 10 mM). A sample was dissolved (45°C, 1500 rpm, one hour) using a heating and vibrating device (Front Lab MyBL-100S, manufactured by AS ONE Corporation). The resulting solution was caused to pass through a hydrophilic PTFE membrane filter having a pore size of 0.45 µm (trade name: Dismic 25HP45AN, manufactured by Advan Tech), and remaining insoluble matters were removed.

### (2-3) Analysis

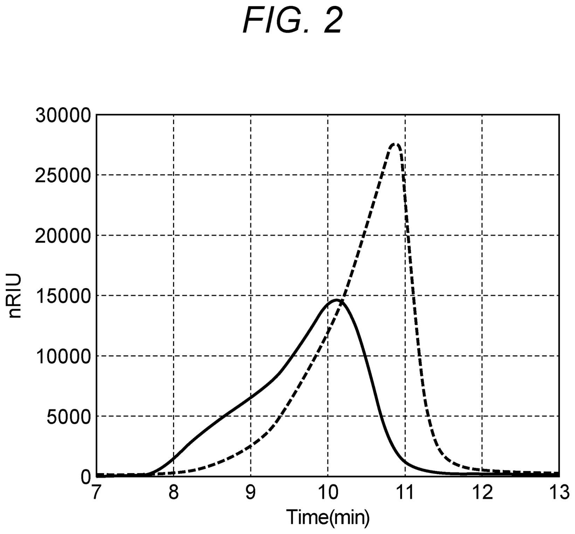

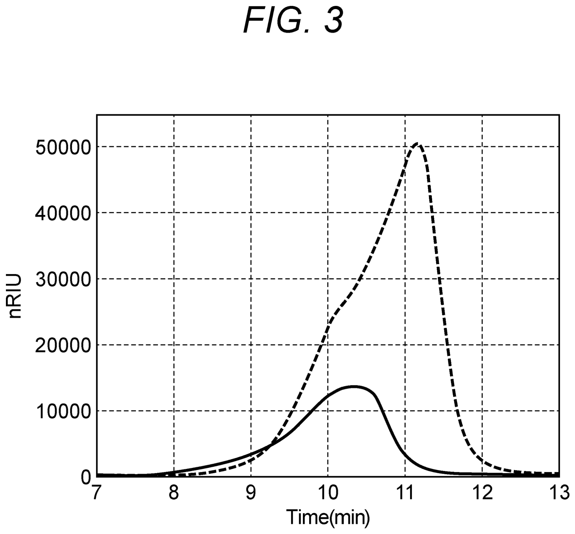

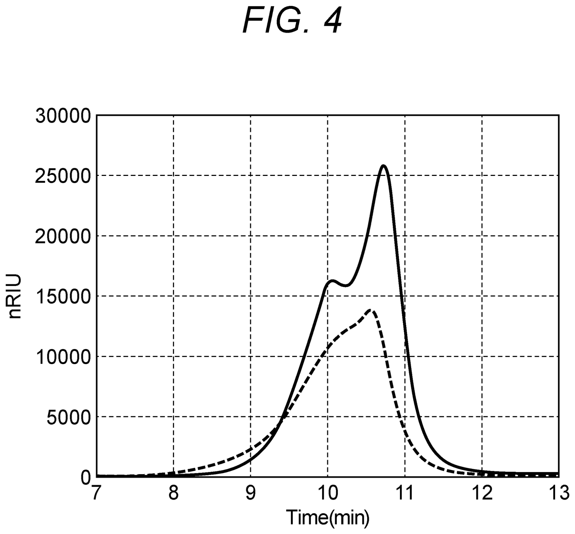

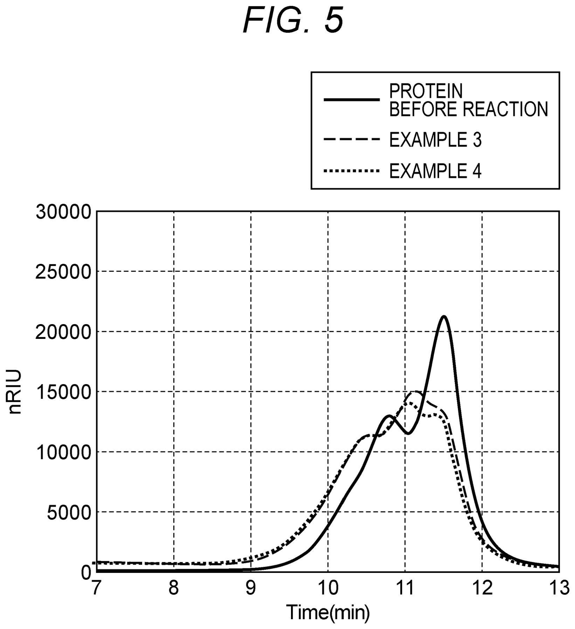

The block copolymer film of Comparative Example 1, the block copolymer powders of Examples 1 to 4, and the protein (PRT2662) used in production of the block copolymer film of Comparative Example 1 and the block copolymer powders of Examples 1 to 4 were subjected to GPC analysis. Results thereof are illustrated in Figs. 2 to 5. As illustrated in Fig. 2, a block copolymer film peak (solid line) of Comparative Example 1 was confirmed at a retention time shorter than that of a protein peak (broken line). As illustrated in Fig. 3, a block copolymer powder peak (solid line) of Example 1 was confirmed at a retention time shorter than that of a protein peak (broken line). As illustrated in Fig. 4, a block copolymer powder peak (solid line) of Example 2 was confirmed at a retention time shorter than that of a protein peak (broken line). As illustrated in Fig. 5, each of block copolymer powder peaks (broken line: Example 3, dotted line: Example 4) of Examples 3 and 4 was confirmed at a retention time shorter than that of a protein peak (solid line). This indicates that both the block copolymer film and the block copolymer powder are those in which dimaleimide PEG is bound to a protein to form a higher molecular weight body.

### 2. Condition study of mechanochemical reaction

Block copolymerization by a mechanochemical method was performed in a similar manner to Example 1 except that conditions of the frequency and the reaction time were changed, and the degree of progress of reaction was evaluated using GPC.

### 2. Change in degree of progress of reaction by frequency

### (1) Preparation of block copolymer (Example 5)

Protein PRT2662 of 27461 Da (100 mg, 3.6 µmol) and dimaleimide PEG of about 20 kDa (73 mg, 3.6 µmol) were put in the 1.5 mL volume ball mill container of the same grinder as used in preparing the block copolymer of Example 1, and then RO water (100 µL) as a swelling solvent was dropped thereto to wet the entire powder. Ten 2 mmφ balls (manufactured by AS ONE Corporation) were put therein and the container was hermetically sealed, and then the pulverizer was vibrated at a frequency of 10 Hz, 20Hz, or 30Hz. After three hours, the reactants were vacuum-dried at room temperature to obtain three types of white powdery block copolymers. The block copolymer powders obtained under the conditions of frequencies of 10 Hz, 20 Hz, and 30 Hz are referred to as Examples 5A, 5B, and 5C, respectively.

### (2) Evaluation

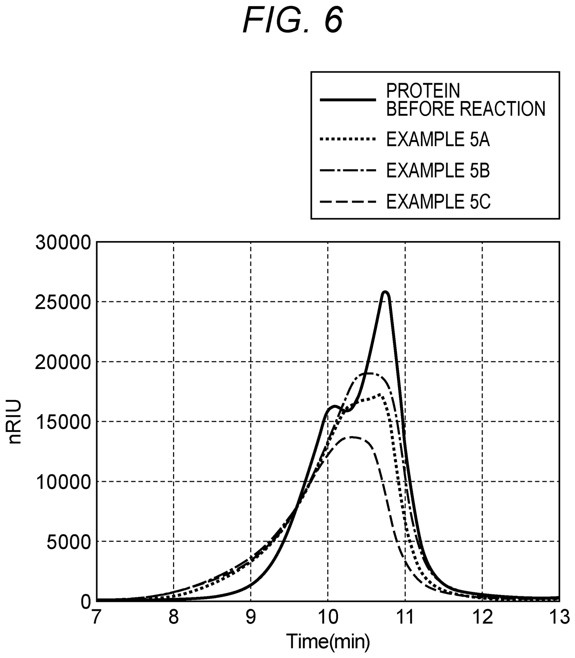

Results of GPC of the block copolymer powders of Examples 5A, 5B, and 5C are illustrated in Fig. 6. In Fig. 6, the solid line indicates a protein peak, the dotted line indicates a block copolymer powder peak of Example 5A obtained by reaction at a frequency of 10 Hz, the alternate long and short dash line indicates a block copolymer powder peak of Example 5B obtained by reaction at a frequency of 20 Hz, and the broken line indicates a block copolymer powder peak of Example 5C obtained by reaction at a frequency of 30 Hz. As illustrated in Fig. 6, regardless of the frequency during the reaction, each of the block copolymer powder peaks was confirmed at a retention time shorter than that of the protein peak. In addition, as the frequency during the reaction increased, a block copolymer powder peak was detected at a shorter retention time. This indicates that, within a certain range, the reaction between the protein and dimaleimide PEG reliably proceeds regardless of the frequency during the reaction, and that, as the frequency is larger within a certain range, the reaction between the protein and dimaleimide PEG is further promoted.

### 3. Change in degree of progress of reaction by reaction time

### (1) Preparation of block copolymer (Example 6)

Protein PRT2662 of 27461 Da (100 mg, 3.6 µmol) and dimaleimide PEG of about 20 kDa (73 mg, 3.6 µmol) were put in the 1.5 mL volume ball mill container of the same grinder as used in preparing the block copolymer of Example 1, and then RO water (100 µL) as a swelling solvent was dropped thereto to wet the entire powder. Ten 2 mmφ balls (manufactured by AS ONE Corporation) were put therein and the container was hermetically sealed, and then the pulverizer was vibrated at a frequency of 30 Hz. After one hour or three hours, the reactants were vacuum-dried at room temperature to obtain two types of white powdery block copolymer powders. The block copolymer powders obtained under the conditions of a reaction time of one hour and three hours are referred to as Examples 6A and 6B, respectively.

### (2) Evaluation

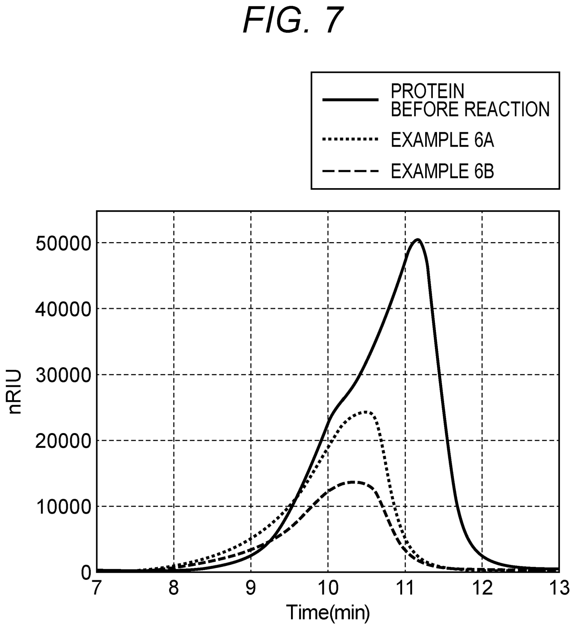

Results of GPC of the block copolymer powders of Examples 6A and 6B are illustrated in Fig. 7. In Fig. 7, the solid line indicates a protein peak, the dotted line indicates a block copolymer powder peak of Example 6A obtained by reaction for a reaction time of one hour, and the broken line indicates a block copolymer powder peak of Example 6B obtained by reaction for a reaction time of three hours. As illustrated in Fig. 7, regardless of the reaction time, each of the block copolymer powder peaks is confirmed at a retention time shorter than that of the protein peak. In addition, as the reaction time was longer, a block copolymer powder peak was detected at a shorter retention time. This indicates that, within a certain range, the reaction between the protein and dimaleimide PEG reliably proceeds regardless of the length of the reaction time, and that, as the reaction time is larger within a certain range, the reaction between the protein and dimaleimide PEG is further promoted.

### 4. Comparison of block copolymer powders

### (1) Preparation of block copolymer

### Comparative Example 2: Preparation of block copolymer powder using solution method and freeze-drying method

Protein PRT2662 of 27461 Da (255 mg, 9.3 µmol) and dimaleimide PEG of about 20 kDa (186 mg, 9.3 µmol) were suspended in DMSO (4.6 mL). Thereafter, while the mixture was heated to 100°C in an oil bath, the mixture was stirred for 15 minutes using a Hercules stirrer (trade name: MODEL HERAXLES/16G, manufactured by Koike Precision Instruments Co., Ltd.) to cause the protein to react with dimaleimide PEG, thereby obtaining a brown and transparent solution in which a reactant thereof was dissolved. Thereafter, about 50 mL of acetone was dropped onto the obtained solution to lower solubility of the solute, and centrifugation (110 rpm, 5 min) was performed to precipitate the solute. Subsequently, the supernatant solvent was discarded, and the resulting solution was shaken well. Thereafter, the same amount of RO water was added thereto, and centrifugation (150 rpm, 10 min) was performed three times in a similar procedure. The same amount of RO water was again added to the obtained gel-like reactant, the mixture was shaken well and dispersed, and then dried with a freeze-drying apparatus using liquid nitrogen for 48 hours or more to obtain a white powdery block copolymer (Comparative Example 2).

### Comparative Example 3: Powderization of block copolymer film prepared using solution method

Protein PRT2662 of 27461 Da (255 mg, 9.3 µmol) and dimaleimide PEG of about 20 kDa (186 mg, 9.3 µmol) were suspended in DMSO (4.6 mL). Thereafter, while the mixture was heated to 100°C in an oil bath, the mixture was stirred for 15 minutes using a Hercules stirrer (trade name: MODEL HERAXLES/16G, manufactured by Koike Precision Instruments Co., Ltd.) to cause the protein to react with dimaleimide PEG, thereby obtaining a brown and transparent solution in which a reactant thereof was dissolved. The obtained solution was thinly applied onto a metal plate to which a release film (manufactured by Teijin Film Solution Co., Ltd., thickness: 38 µm) was attached using a doctor blade (manufactured by Imoto machinery Co., Ltd., coating width: 80 mm, gap: 400 µm) to obtain a coating film. Subsequently, the coating film was dried at 60°C for two hours or more using a blown constant temperature thermostat, and furthermore, the solvent was removed at 80°C for 15 hours using a vacuum oven to obtain a brown and transparent film. This film was put in a 5 mL volume ball mill container (manufactured by Verder Scientific Co., Ltd.) together with two 10 mmφ pulverizing balls (manufactured by Verder Scientific Co., Ltd.), and the ball mill (trade name: MM400, manufactured by Vader Scientific Co., Ltd.) was vibrated at a frequency of 30 Hz and pulverized. After 30 minutes, the pulverized product was vacuum-dried at room temperature to obtain a brown and transparent powdery block copolymer (Comparative Example 3).

### Comparative Example 4: Powderization of block copolymer gel prepared using solution method

Protein PRT2662 of 27461 Da (255 mg, 9.3 µmol) and dimaleimide PEG of about 20 kDa (186 mg, 9.3 µmol) were suspended in DMSO (4.6 mL). Thereafter, while the mixture was heated to 100°C in an oil bath, the mixture was stirred for 15 minutes using a Hercules stirrer (trade name: MODEL HERAXLES/16G, manufactured by Koike Precision Instruments Co., Ltd.) to cause the protein to react with dimaleimide PEG, thereby obtaining a brown and transparent solution in which a reactant thereof was dissolved. This solution was poured into a mold formed of a metal plate, a release film (Teijin Film Solution Co., Ltd., thickness: 38 µm), and a silicon sheet cut into a mouth shape (manufactured by TOGAWA RUBBER CO., LTD.), and allowed to stand at room temperature for 15 hours or more. The mold into which the solution had been poured was shaken in normal temperature ethanol for three hours using a shaker, and then washed twice with RO water at 60°C for ten minutes to obtain a colorless and transparent block copolymer gel. The obtained gel was dried at 60°C for two hours or more using a blown constant temperature thermostat, and furthermore, the solvent was removed at 80°C for 15 hours using a vacuum oven to obtain a brown and transparent solid block copolymer. This solid was put in a 5 mL volume ball mill container (manufactured by Verder Scientific Co., Ltd.) together with two 10 mmφ pulverizing balls (manufactured by Verder Scientific Co., Ltd.), and the ball mill (trade name: MM400 manufactured by Vader Scientific Co., Ltd.) was vibrated at a frequency of 30 Hz and pulverized. After 30 minutes, the pulverized product was vacuum-dried at room temperature to obtain a light brown powdery block copolymer (Comparative Example 4).

### Example 7: Preparation of block copolymer powder using mechanochemical method and freeze-drying method

Protein PRT2662 of 27461 Da (300 mg, 10.8 µmol) and dimaleimide PEG of about 20 kDa (216 mg, 10.8 µmol) were put in the 1.5 mL volume ball mill container of the same grinder as used in preparing the block copolymer of Example 1, and then RO water (300 µL) as a swelling solvent was dropped thereto to wet the entire powder. Ten 2 mmφ balls (manufactured by AS ONE Corporation) were put therein and the container was hermetically sealed, and then the pulverizer was vibrated at a frequency of 30 Hz to cause the protein to react with the dimaleimide PEG. After three hours, the reactants were vacuum-dried at room temperature to obtain a white powdery block copolymer (Example 7).

### (2) Evaluation

### (2-1) State of fine powder

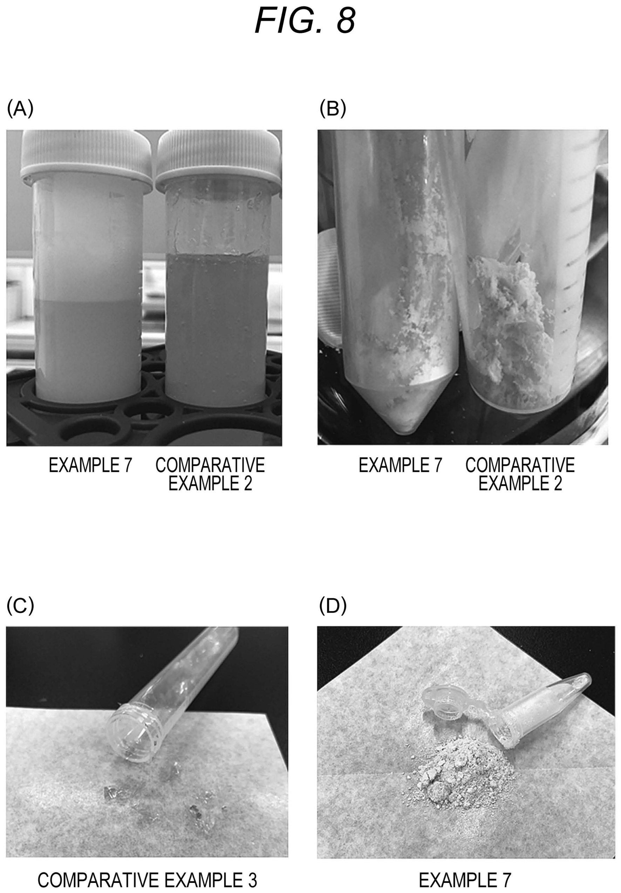

The block copolymers of Comparative Examples 2 to 4 and Example 7 were observed and compared with images. Fig. 8(A) is a photograph illustrating a state in which the products immediately before freeze-drying in Example 7 (left) and Comparative Example 2 (right) were dispersed in RO water, and Fig. 8(B) is a photograph illustrating appearances of powders immediately after freeze-drying in Example 7 (left) and Comparative Example 2 (right). According to Fig. 8(A), it is observed that a powder is well dispersed in the block copolymer of Example 7 to form a uniform white suspension, whereas a gel adheres to a wall surface in the block copolymer of Comparative Example 2. In addition, according to Fig. 8(B), the block copolymer of Example 7 was a clear fine powder, whereas the block copolymer of Comparative Example 2 was a large lump. Fig. 8(C) is a photograph illustrating a state after the block copolymer film in Comparative Example 3 was pulverized. It is considered that since the block copolymer film of Comparative Example 3 was soft, a force of the pulverizing balls was not reflected, and a fine powder could not be obtained. Fig. 8(D) is a photograph illustrating a state after the block copolymer gel of Comparative Example 4 was pulverized. The obtained block copolymer powder was slightly colored as compared with the block copolymer powder of Example 7.

### (2-2) Particle size distribution measurement

Each of the block copolymer powder of Example 7 and the block copolymer powder of Comparative Example 4 was uniformly dispersed on a glass plate by suction with a vacuum chamber, and then measured five times using a particle size analyzer for analysis of both a dry image and a wet image (trade name: DW-200nano, manufactured by JASCO International Corporation), a projection image was photographed with a 10 million pixel camera, and the obtained projection image was analyzed using image analysis software.

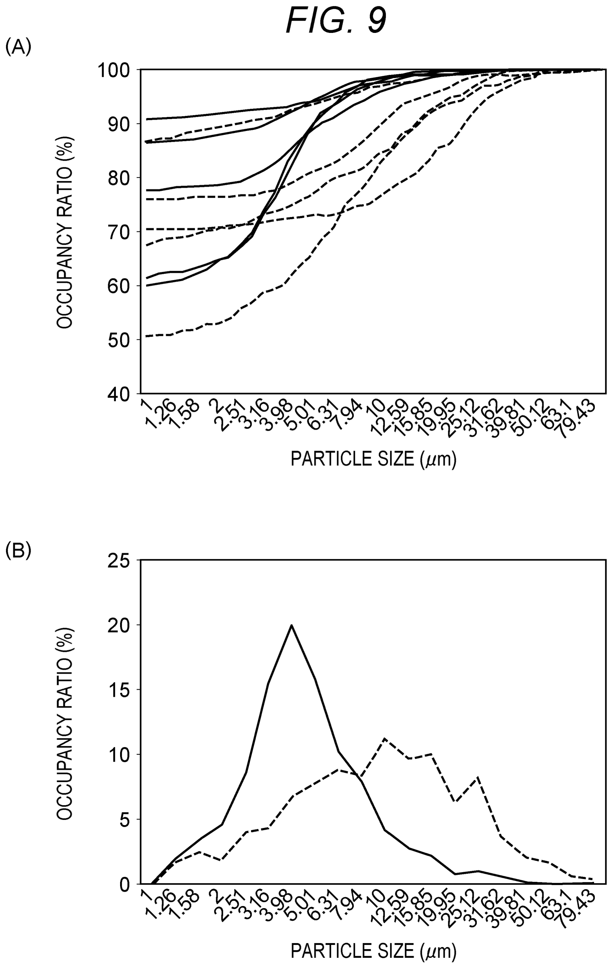

Fig. 9 is a graph illustrating particle size distributions of block copolymer powders of Example 7 and Comparative Example 4. In Fig. 9(A), the solid line indicates a particle size of the block copolymer powder of Example 7 and an occupancy ratio (accumulation) thereof, and the broken line indicates a particle size of the block copolymer powder of Comparative Example 4 and an occupancy ratio (accumulation) thereof. In Fig. 9(B), the solid line indicates a particle size of the block copolymer powder of Example 7 and an occupancy ratio thereof, and the broken line indicates a particle size of the block copolymer powder of Comparative Example 4 and an occupancy ratio thereof. Note that, in Fig. 9(B), particles having a particle size of 1 µm or less, for which accurate measurement is difficult, were excluded. From the graph of Fig. 9(A), it was found that about 15% of the block copolymer powder of Comparative Example 4 had a particle size of more than 10 µm, whereas only about 3% of the block copolymer powder of Example 7 had a particle size of more than 10 µm. From the graph of Fig. 9(B), it was found that in the block copolymer powder of Comparative Example 4, a gentle peak (standard deviation: 6.1) was drawn at a position of 10 to 20 µm, whereas in the block copolymer powder of Example 7, a peak (standard deviation: 3.6) having a narrower peak width was drawn at a position of about 4 µm, there were many particles having a smaller particle size, and the particle sizes were uniformly distributed.

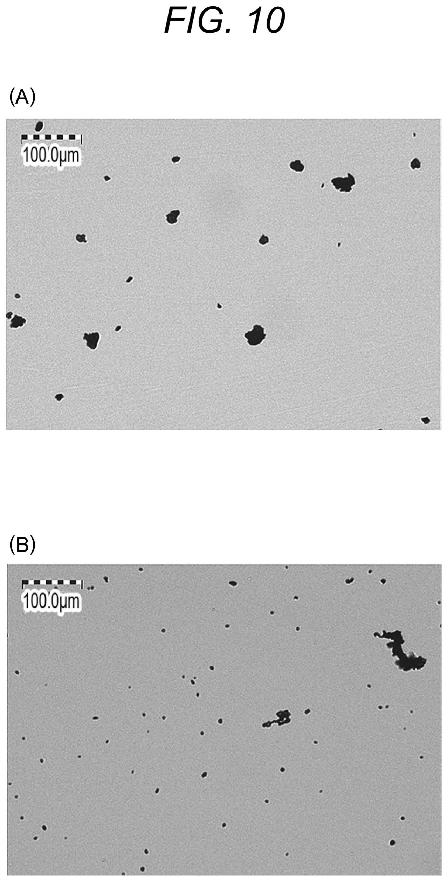

Fig. 10(A) is a micrograph of the block copolymer powder of Comparative Example 4, and Fig. 10(B) is a micrograph of the block copolymer powder of Example 7. It was observed also from the appearance that the block copolymer powder of Example 7 had many smaller particles than the block copolymer powder of Comparative Example 4, and had a uniform particle size distribution.

### 5. Comparison of physical properties

Each of the block copolymers obtained above was processed by a method well known to those skilled in the art, and then physical properties of the processed products were compared.

### (1) Comparison of residual amount of organic solvent

The amount of the remaining organic solvent in the block copolymer film of Comparative Example 1 was evaluated using gas chromatography (trade name: GC-2010 Plus, manufactured by Shimadzu Corporation). Specifically, 43 mg of the block copolymer film of Comparative Example 1 was cut off to prepare a sample. The sample was immersed in methanol (10 mL) for 24 hours or more, and then the temperature of the methanol solution was increased to 240°C at 10°C/min and kept at this temperature for two minutes to volatilize the solution, and the DMSO residual amount in the sample was measured. Measurement conditions are presented in Table 3. The measurement was performed twice, and an average value of the DMSO residual amounts was recorded.

**[Table 3]**

| Column | Rxi-624 sil MS (manufactured by GL Sciences Inc., length: 30 m, inner diameter: 0.25 mm) |
|---|---|
| Pressure | 140 kPa |
| Total flow rate | 15.6 mL/min |
| Injection mode | Split (5 : 1) |
| Carrier gas | Helium gas |

Measurement results of gas chromatography are presented in Table 4. As is apparent from Table 4, the DMSO residual amount in the block copolymer film of Comparative Example 1 was 0.106 wt% on average. In a block copolymer obtained by a solution method, it is necessary to dissolve a protein using an organic solvent such as DMSO at the time of preparation of the block copolymer, and thus the organic solvent tends to remain in the block copolymer. On the other hand, in a block copolymer obtained by a mechanochemical method, it is not necessary to dissolve a protein in an organic solvent at the time of preparation of the block copolymer, and thus it is considered that the organic solvent does not remain in the block copolymer.

**[Table 4]**

| No. | Weight of sample [g] | GC quantification result [µg/mL] | Weight of DMSO eluted into methanol [g] | Amount of residual DMSO in sample [ppm] | Average [wt%] |
|---|---|---|---|---|---|
| 1 | 0.0433 | 4.515 | 0.000045 | 1042.7 | 0.106 |
| 2 | 0.0433 | 4.679 | 0.000047 | 1080.6 | |

### (2) Water-dispersibility test

In an Eppendorf tube, 0.1 g of the block copolymer powder of Example 7 was put, and then RO water was added thereto so as to have a solid content concentration of 8%. Thereafter, the tube was gently shaken by hand to prepare a block copolymer aqueous dispersion (Example 8). In addition, a block copolymer aqueous dispersion (Comparative Example 5) was prepared in a similar manner to that described above except for using the block copolymer powder of Comparative Example 4 in place of the block copolymer powder of Example 7. Subsequently, in order to confirm a dispersion state of each of the block copolymers in the obtained block copolymer aqueous dispersion of Example 8 and the obtained block copolymer aqueous dispersion of Comparative Example 5, a turbidity test was performed.

### <Turbidity test>

Turbidity of the dispersion was determined by turbidity measurement according to JIS K0101 "Industrial water testing method". RO water (manufactured by UNIMAT LIFE, R.O.PureRainbow) was added to each of the block copolymer powder of Comparative Example 4 and the block copolymer powder of Example 7 so as to have a solid content concentration of 0.5, 1.0, 1.5, or 2.0%, and the mixture was put in a cell for measuring absorbance (Kartell, disposable cell 1.5 mL, 2-478-11), shaken well, and then allowed to stand for one minute, and absorbance was measured with an ultraviolet-visible spectrophotometer (Shimadzu Corporation, UV-2600). In this experimental system, since precipitation occurs in the solution, absorbance at a position of 15 mm from a lower part of the cell was analyzed.

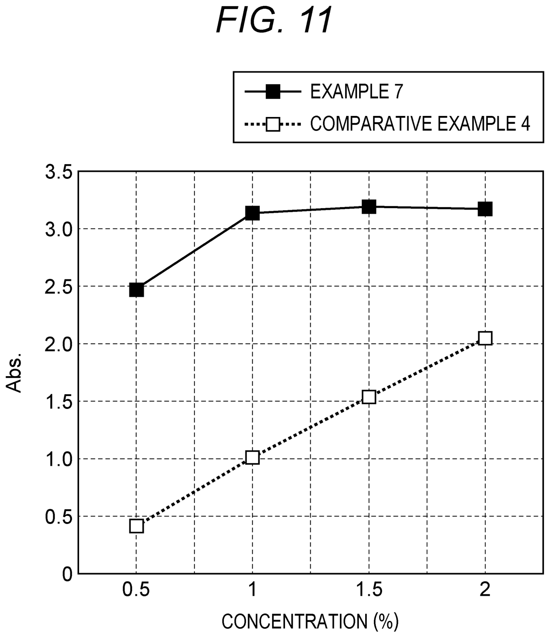

Fig. 11 illustrates a diagram in which the block copolymer powders of Comparative Example 4 and Example 7 were dispersed in RO water at a concentration of 0.5 to 2.0%, and turbidities thereof were compared by absorption analysis. The solid line indicates results of Example 7, and the dotted line indicates results of Comparative Example 4. In Comparative Example 4, precipitation quickly occurred after shaking at any concentration, and therefore coloring was hardly observed in a portion through which a laser beam passed, and a light absorption value was also low. On the other hand, in Example 7, cloudiness was well maintained even after a lapse of a certain time after shaking, and an absorbance value was also higher than that of the solution-based reactant. However, in Example 7, there was no change in absorbance at a certain concentration or more. From the above results, absorbance (Abs.) of an aqueous dispersion obtained by dispersing a block copolymer powder obtained by a mechanochemical treatment in RO water at a concentration of 1% is preferably more than 22.0.

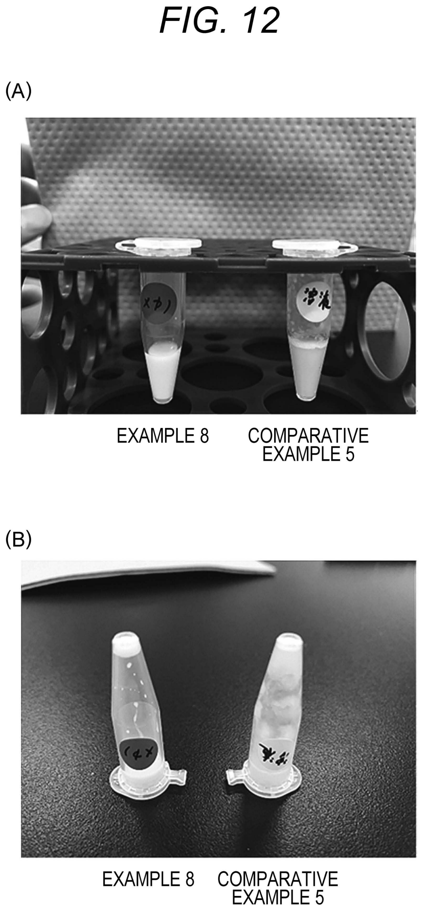

Fig. 12(A) is a photograph illustrating a dispersion state of each of block copolymers in a block copolymer aqueous dispersion of Example 8 (left) and a block copolymer aqueous dispersion of Comparative Example 5 (right). Fig. 12(B) is a photograph illustrating a state of each of the aqueous dispersions when each Eppendorf tube illustrated in Fig. 12(A) was placed upside down. As is apparent from Figs. 12(A) and 12(B), the aqueous dispersion of Example 8 was dispersed so as to be uniformly cloudy, whereas the aqueous dispersion of Comparative Example 5 had gel-like insolubles adhering to a wall of the tube. This indicates that the block copolymer obtained by the mechanochemical method has higher water-dispersibility than the block copolymer obtained by the solution method.

### (3) Preparation of coating film

The block copolymer aqueous dispersion of Example 8 was thinly applied to a metal plate to which a release film was attached, put in a blown constant temperature thermostat, and dried at 60°C for two hours to obtain a block copolymer coating film (Example 9). In addition, a block copolymer coating film (Comparative Example 6) was obtained in a similar manner to that described above except for using the block copolymer aqueous dispersion of Comparative Example 5 in place of the block copolymer aqueous dispersion of Example 8. Subsequently, properties of the obtained block copolymer coating film of Example 9 and the obtained block copolymer coating film of Comparative Example 6 were visually confirmed.

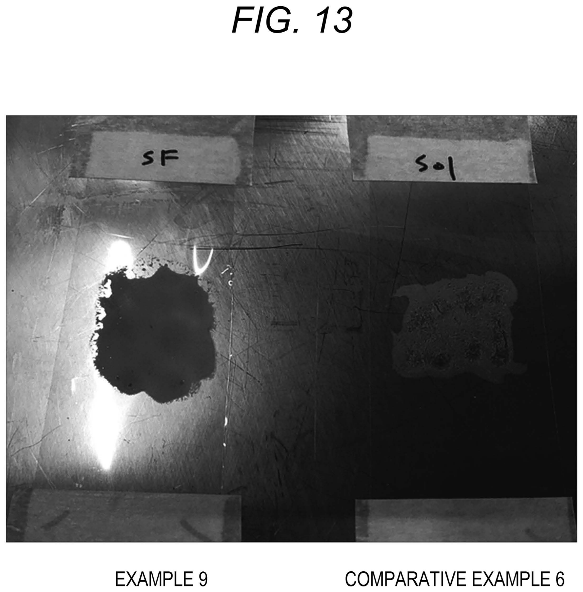

Fig. 13 (left) is a photograph illustrating the properties of the block copolymer coating film of Example 9, and Fig. 13 (right) is a photograph illustrating the properties of the block copolymer coating film of Comparative Example 6. As is apparent from Fig. 13 (left) and Fig. 13 (right), the coating film of Example 9 had a uniform thickness, whereas the coating film of Comparative Example 6 had a non-uniform thickness and had portions where no film was formed. This indicates that the block copolymer aqueous dispersion obtained by the mechanochemical method can be used as a coating liquid capable of forming a coating film having a uniform thickness, unlike the block copolymer aqueous dispersion obtained by the solution method.

### (4) Preparation of resin

A mold release agent DAIFREE (trade name: MS-600 manufactured by Daikin Industries, Ltd.) was thinly baked inside a 1.5 mm × 3.5 mm mold (manufactured by Global Machine Co., Ltd.) to perform an adhesion preventing treatment. 2 g of the block copolymer powder of Example 7 was put in the mold that had been subjected to the adhesion prevention treatment at room temperature, and a metal lid connected to a pressurizing jack was closed. Thereafter, using a pressure molding machine (trade name: NT-100H, manufactured by NPa System Co., Ltd.), a pressure of 15 MPa was applied, heating was performed up to 130°C, and the temperature and the pressure were kept constant after the temperature reached 130°C, whereby heating and molding were performed. After three minutes, the molded product was cooled to room temperature and the pressure was removed, and then the molded body was taken out to obtain a brown and transparent block copolymer molded body (resin, Example 10). In addition, a block copolymer molded body (Comparative Example 7) was molded in a similar manner to that described above except for using the block copolymer powder of Comparative Example 4 in place of the block copolymer powder of Example 7.

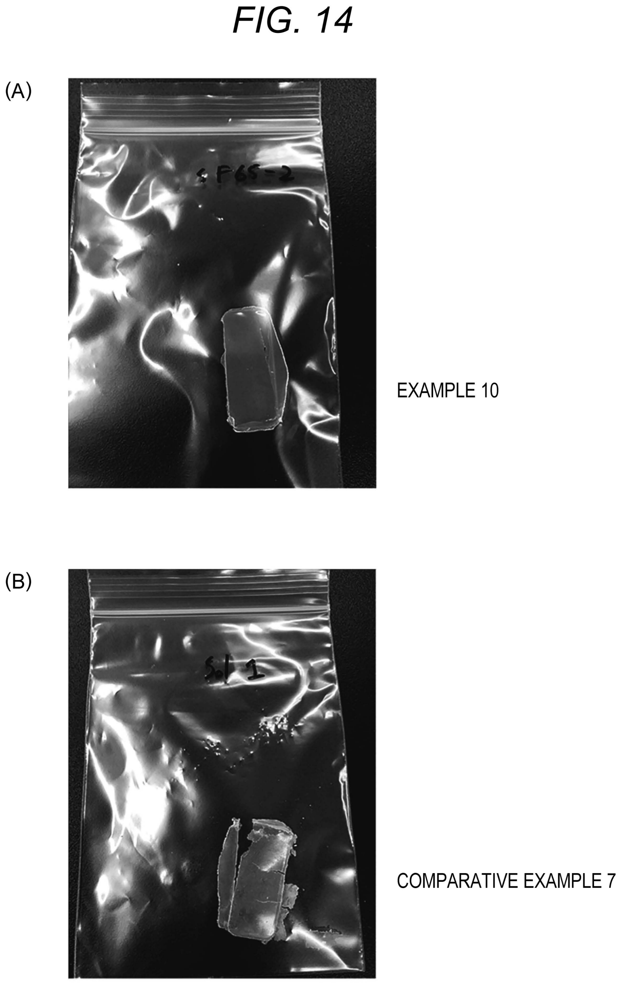

Fig. 14(A) is a photograph illustrating the properties of the block copolymer molded body of Example 10, and Fig. 14(B) is a photograph illustrating the properties of the block copolymer molded body of Comparative Example 7. As is apparent from Figs. 14(A) and 14(B), cloudiness and cracking were not observed in the block copolymer molded body of Example 10, whereas the block copolymer molded body of Comparative Example 7 had much cloudiness and low strength to such an extent that the block copolymer molded body was easily cracked. This indicates that the block copolymer molded body obtained by the mechanochemical method has less cloudiness and higher strength than the block copolymer molded body obtained by the solution method.

### (5) Preparation of water-dispersible adhesive

The block copolymer aqueous dispersion of Example 8 was thinly applied to a region of 15 mm × 15 mm at one end of wood (made of Japanese cypress, 1.5 mm × 40 mm × 2 mm), and another piece of wood of the same size was superimposed thereon. Thereafter, the two superimposed pieces of wood were fixed with a clip, put in a blown constant temperature thermostat, and dried at 60°C for 48 hours to bond the two pieces of wood to each other. In addition, two pieces of wood were bonded to each other in a similar manner to that described above except for using the block copolymer aqueous dispersion of Comparative Example 5 in place of the block copolymer aqueous dispersion of Example 8.

### (6) Tensile test of bonded wood

The wood bonded with the block copolymer aqueous dispersion of Example 8 and the wood bonded with the block copolymer aqueous dispersion of Comparative Example 5 were each stored under an environment of room temperature of 20°C and a humidity of 65% for 24 hours or more, then set in a tensile tester (trade name: AG-X plus50kN, manufactured by Shimadzu Corporation), and was repeatedly subjected to a tensile test ten times. Note that the tensile test was performed under conditions of a load cell: 50 kN, a distance between grips: 57.5 mm, and a tensile speed: 10 mm/min.

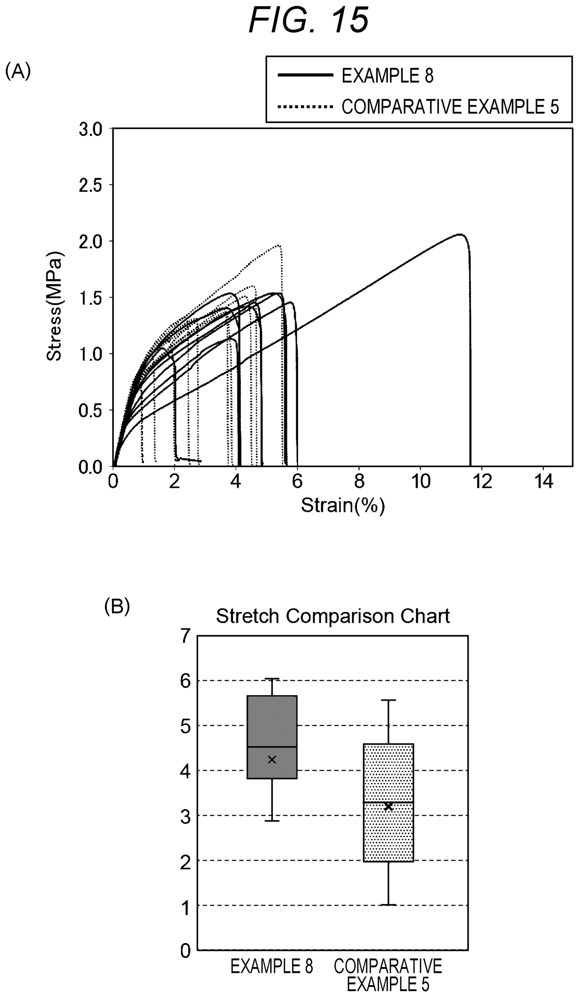

Fig. 15(A) is a graph illustrating results of the tensile test. In Fig. 15(A), the vertical axis represents stress, the horizontal axis represents elongation, the solid line indicates results of the tensile test of the wood bonded using the block copolymer aqueous dispersion of Example 8, and the dotted line indicates results of the tensile test of the wood bonded using the block copolymer aqueous dispersion of Comparative Example 5. As illustrated in Fig. 15(A), breaking elongation of the wood bonded with the block copolymer aqueous dispersion of Example 8 was about 11.8%, whereas breaking elongation of the wood bonded with the block copolymer aqueous dispersion of Comparative Example 5 was about 5.5%. Fig. 15(B) is a box plot illustrating results of elongation illustrated in Fig. 15(A). As illustrated in Fig. 15(B), the wood bonded with the block copolymer aqueous dispersion of Example 8 (left) had an average elongation of 4.7%, whereas the wood bonded with the block copolymer aqueous dispersion of Comparative Example 5 (right) had an average elongation of 3.3%. These indicate that the block copolymer aqueous dispersion of Example 8 can be used as an adhesive capable of bonding wood with higher strength than the block copolymer aqueous dispersion of Comparative Example 5.

### 6. Preparation of prepolymer

An excessive amount of polyethylene glycol (PEG) having two maleimide groups was added to a protein having two thiol groups and caused to react therewith to prepare a prepolymer having reactive maleimide groups at both ends. To the obtained prepolymer, a curing agent having two thiol groups was further added in an equimolar amount with the prepolymer and caused to react therewith to prepare a finished polymer. The degrees of progress of these reactions were evaluated using GPC. The reactions are described below.

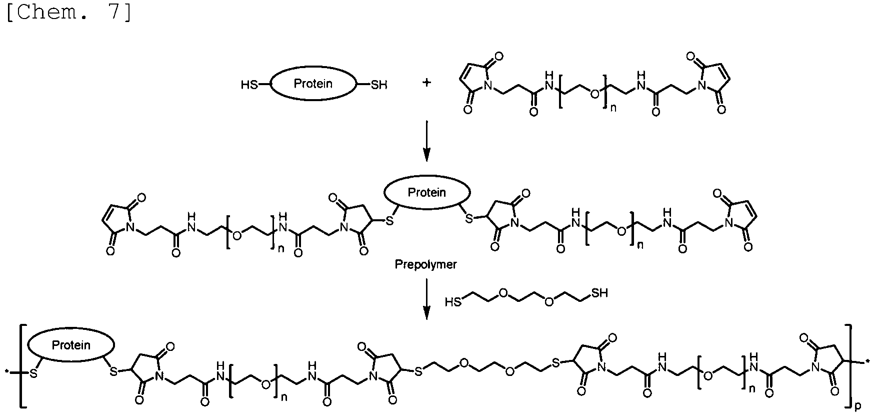

### (2) Prepolymer synthesis by mechanochemical method

### (Example 11)

Protein PRT2662 of 27461 Da (211 mg, 7.7 µmol) and dimaleimide PEG of about 20 kDa (307 mg, 15.4 µmol) were put in a 5 mL volume ball mill container of the same grinder as used in preparing the block copolymer of Example 1, and then RO water (300 µL) as a swelling solvent was dropped thereto to wet the entire powder. Two 10 mmφ pulverizing balls (manufactured by Verder Scientific Co., Ltd.) were put therein and the container was hermetically sealed, and then the pulverizer was vibrated at a frequency of 30 Hz to cause the protein to react with the dimaleimide PEG. After three hours, the reactants were vacuum-dried at room temperature to obtain a white powdery block copolymer prepolymer (Example 11A).

In addition, 80 mg of the block copolymer prepolymer of Example 11A was suspended in DMSO (909 mL), and the resulting suspension was stirred for 15 minutes using a Hercules stirrer (trade name: MODEL HERAXLES/16G, manufactured by Koike Precision Instruments Co., Ltd.) while being heated to 70°C in an oil bath, and cooled to room temperature. After cooling, 2,2'-(ethylenedioxy) diethanethiol (1.4 mg, 7.7 µmol) was added to the suspension, and then the mixture was stirred for 30 minutes to cause the block copolymer prepolymer to react with diethanethiol, thereby obtaining a solution in which a brown and transparent block copolymer finished polymer was dissolved. The obtained block copolymer finished polymer solution was applied onto a release film (Teijin Film Solution Co., Ltd., thickness: 38 µm) attached onto a surface of a metal plate using a doctor blade (manufactured by Imoto machinery Co., Ltd.) so as to have a coating width of 80 mm and a gap of 400 µm. The applied metal plate was dried at 60°C for two hours or more using a blown constant temperature thermostat, and furthermore, the solvent was removed at 80°C for 15 hours using a vacuum oven to obtain a brown and transparent block copolymer finished polymer film (Comparative Example 11B).

### (3) GPC analysis

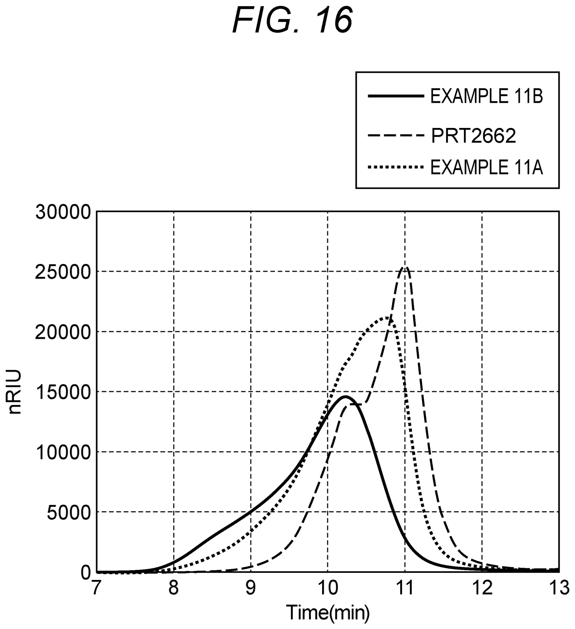

Fig. 16 is a graph illustrating results of GPC of protein PRT2662 (broken line), Example 11A (dotted line), and Example 11B (solid line). In both Example 11A and Example 11, since the retention time was shortened as the reaction proceeded, it was suggested that the reaction could be controlled as designed such that the molecular weight was increased as the reaction proceeded.

### 7. Mechanochemical polymerization using mixer mill

Whether a block copolymer can be prepared by causing a protein having one or more thiol groups to react with polyethylene glycol (PEG) having one or more maleic acid ester groups under mechanochemical conditions using a mixer mill was evaluated using gel permeation chromatography (GPC) analysis. In the reaction, a test kneader (Labo Plast Mill 3S150, manufactured by Toyo Seiki Seisaku-sho, Ltd.) equipped with a roller mixer model R60 (manufactured by Toyo Seiki Seisaku-sho, Ltd., blade shape: roller type) was used as a different direction rotating roller mixer.

An outline of the reaction is described below. The block copolymer as a product has a repeating structure of a unit structure represented by the following formula bound at the asterisk (*).

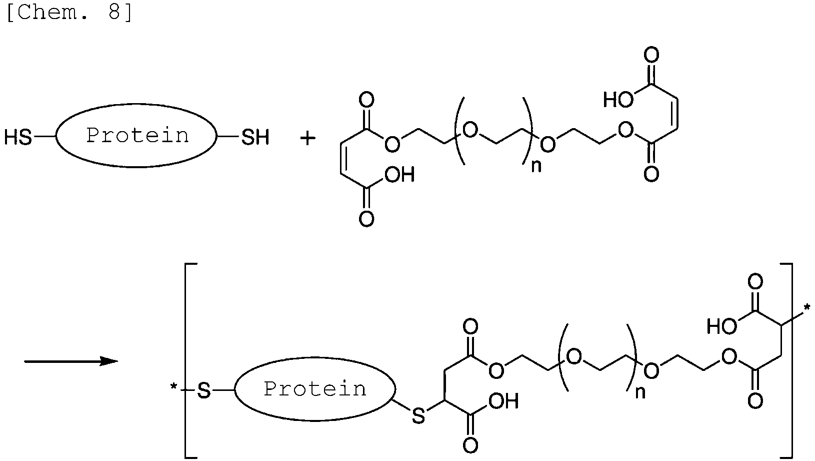

### (Example 12)

Protein PRT2882 of 10kDa (15 g, 16 mmol), maleic acid esterified polyethylene glycol of 11kDa (15 g, 1.4 mmol), and a reducing agent dithiothreitol (430 mg, 2.8 mmol) are each weighed and mixed well. Before the reaction, a temperature in a feed chamber (60 mL) of the mixer mill was adjusted to 85°C. A mixture containing recombinant protein PRT2882 of 100 kDa was put in the feed chamber in a divided manner while a blade of the mixer mill was inched (10 rpm), and then triethylamine (1.63 mL, 11.7 mmol) was dropped thereto. Inching (10 rpm) was performed until the reactants were homogenized. After it was confirmed that both torque and temperature were stable, a milling procedure was initiated, and the reactants were kneaded continuously (50 rpm, 85°C, one hour) to obtain a product as a brown solid. After the reaction, a part of the product was scraped off, and was subjected to GPC measurement.

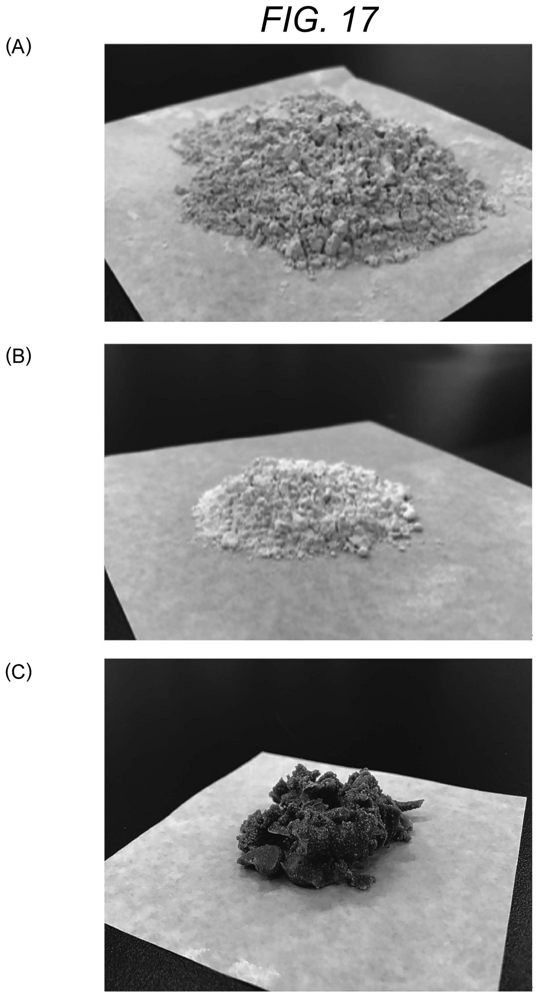

An appearance of the obtained product is illustrated in Fig. 17 together with unmodified protein PRT2882 and maleic acid esterified polyethylene glycol. Fig. 17, Fig. 17(A) is a photograph illustrating an appearance of unmodified protein PRT2882, Fig. 17(B) is a photograph illustrating an appearance of maleic acid esterified polyethylene glycol, and Fig. 17(C) is a photograph illustrating an appearance of the product.

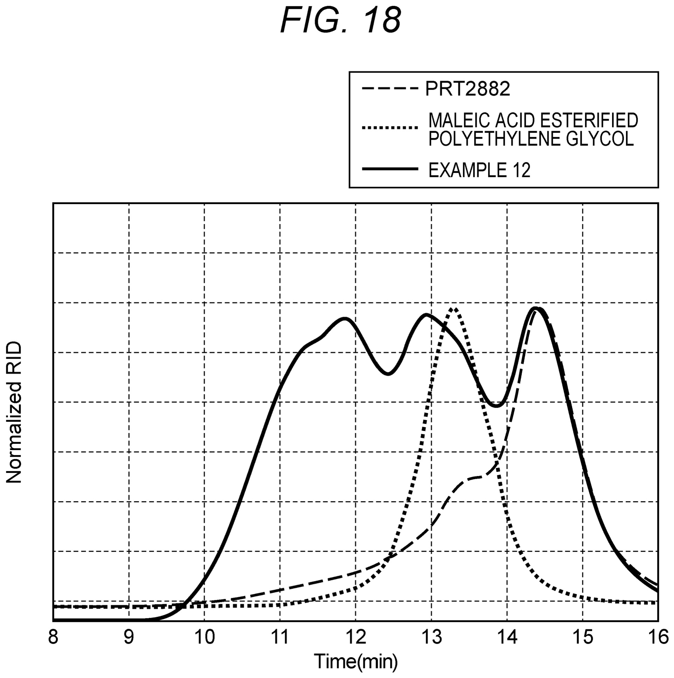

The obtained product was subjected to GPC analysis by the above-described method. Results thereof are illustrated in Fig. 18. In Fig. 18, the broken line is a chromatogram of unmodified protein PRT2882, the dotted line is a chromatogram of maleic acid esterified polyethylene glycol, and the solid line is a chromatogram of the product. In the chromatogram of the product, a peak appeared at a position with a shorter retention time than the peaks of the raw materials, and thus it is considered that the reaction proceeded to generate a block copolymer.

### 8. Reaction of amine with aldehyde

### (1) Synthesis of dialdehyde PEG

### Step 1: Nucleophilic substitution reaction

Under ambient conditions, polyethylene glycol 10000 (20 g, 2.00 mmol, also referred to as "PEG10K") was added to a mixture of sodium hydroxide (480 mg, 12.00 mmol) and water (48 µL), and the resulting mixture was heated to 80°C with stirring. After PEG10K had fully melted to a homogenous suspension, chloroacetaldehyde dimethyl acetal (2.05 mL, 9.00 mmol) was dropped to the molten suspension at 80°C with stirring and stirring was continued for two hours. Thereafter, second time chloroacetaldehyde dimethyl acetal (2.05 mL, 9.00 mmol) was added to the molten suspension, and stirring was continued for another two hours. After cooling, the reaction mixture was dissolved in DCM (150 mL) and filtered by gravity filtration, and salts were removed. The filtrate was concentrated in vacuo, the product was poured into hexane, and the mixture was triturated with rapid stirring. The product was isolated by vacuum filtration and washed with hexane. The collected solid was partially dried on a filter, then transferred to a round bottom flask, and dried under reduced pressure. First, the solid was dried using a rotary evaporator (45°C) until it was visibly dry, and then dried thoroughly using an oil pump vacuum line to remove residual solvent and moisture, thereby obtaining bis(dimethylacetal) of PEG10K as a colorless amorphous solid.

### Step 2: Hydrolysis of acetal with acid catalyst

Under ambient conditions, bis(dimethylacetal) (18 g, 1.77 mmol) of PEG10K was suspended in 1 M hydrochloric acid (20 mL, 20.0 mmol) at room temperature and stirred rapidly, the reaction temperature was raised to 80°C, and stirring was continued for three hours until the reaction was completed. The reactants were cooled and the product was extracted with DCM (4 x 50 mL). The organic layers were combined, dried with anhydrous sodium sulfate, filtered, concentrated under reduced pressure, and triturated by pouring the product into ice-cold hexane (200 mL) with rapid stirring. The product was isolated by filtration under reduced pressure and washed with hexane. The collected solid was partially dried on a filter, then transferred to a round bottom flask, and first dried under reduced pressure using a rotary evaporator (45°C) until it was visibly dry, then dried thoroughly using an oil pump vacuum line to remove residual solvent and moisture, thereby obtaining dialdehyde of PEG10K (PEG-I) as a white amorphous solid.

### (3-1) Synthesis of block copolymer in liquid phase

Protein PRT3463 of 10763 kDa (518 mg, 48 µmol) and dialdehyde PEG of about 10 kDa (482 mg, 48 µmol) were suspended in DMSO (8.18 mL). Thereafter, while the mixture was heated to 100°C in an oil bath, the mixture was stirred for five hours using a Hercules stirrer (trade name: MODEL HERAXLES/16G, manufactured by Koike Precision Instruments Co., Ltd.) to cause the protein to react with dialdehyde PEG, thereby obtaining a brown and transparent solution in which a reactant thereof was dissolved. Thereafter, the obtained solution was thinly applied to a metal plate to which a release film (manufactured by Teijin Film Solution Co., Ltd., thickness: 38 µm) was attached using a doctor blade (manufactured by Imoto machinery Co., Ltd., coating width: 80 mm, gap: 400 µm) to obtain a coating film. Subsequently, the coating film was dried at 60°C for two hours or more using a blown constant temperature thermostat, and furthermore, the solvent was removed at 80°C for 15 hours using a vacuum oven to obtain a brown and transparent film-shaped block copolymer (Comparative Example 8).

### (3-2) Synthesis of block copolymer by mechanochemical

PRT3463 protein of 10763 kDa (518 mg, 48 µmol, SEQ ID NO: 11), dialdehyde PEG of about 10 kDa (482 mg, 48 µmol), and THF (0 to 5 wt%) were each weighed and mixed well beforehand. These were put in a ball mill container (manufactured by Verder Scientific Co., Ltd.), balls (manufactured by AS ONE Corporation, Φ = 2 mm, ten balls) were put therein, and the container was hermetically sealed. Thereafter, a reaction was caused at a frequency of 30 Hz using a ball mill (Verder Scientific Co., Ltd., trade name: MM400). After one hour, the reactants were dried under reduced pressure at room temperature to obtain a white powdery block copolymer (Example 13).

### (4) GPC analysis

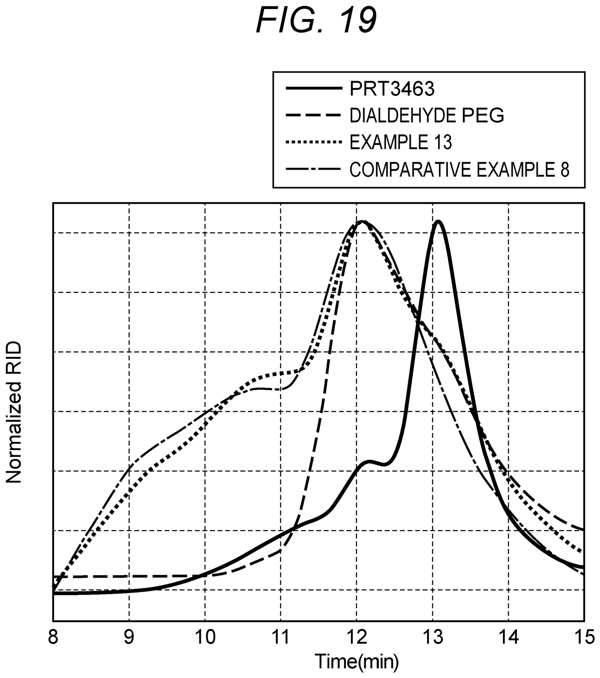

The reaction product obtained in the above (3-1) or (3-2) was subjected to GPC analysis. Results of GPC are illustrated in Fig. 19. Under any of the reaction conditions, a peak indicating a larger molecular weight than the peaks of protein PRT3463 and dialdehyde PEG before the reaction was observed. This result suggests that it is possible to produce a block copolymer by causing an amine to react with an aldehyde by a mechanochemical reaction.

### (5) Comparison of DMSO solubility

The film obtained in the above (3-1) was made into a powder form as fine as possible using a pestle and a mortar in advance. The powder obtained in the above (3-2) was used as it was. A DMSO solution (protein PRT3463: 50 mg, DMSO: 864 µL) containing 5% of protein powder was prepared, heated in an oil bath at 85°C for 15 minutes, and visually observed.

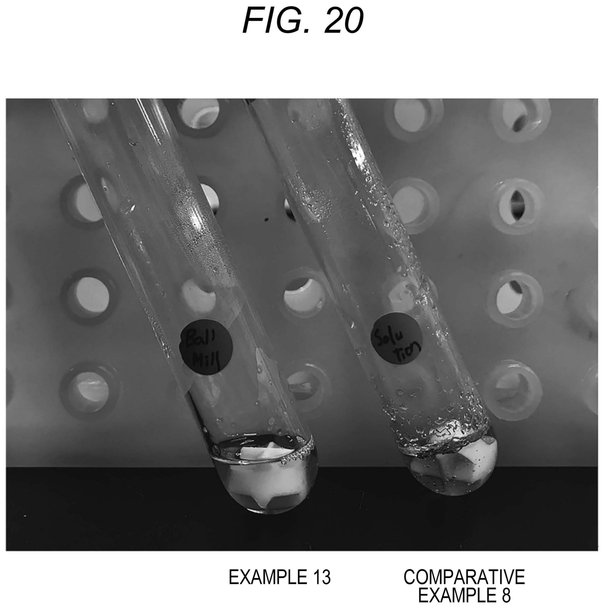

As a result, as illustrated in Fig. 20, the powder of Example 13 was neatly dissolved in DMSO, whereas the powder derived from the film of Comparative Example 8 was in a gel state. The powder of Example 13 has a small particle size and a loosened multidimensional structure of proteins, and thus the powder can be easily dissolved, whereas the powder derived from the film of Comparative Example 8 includes a particle having a large particle size and has many multidimensional structures of proteins formed at the time of film formation, and thus is considered to have a lowered solubility. From these results, it was confirmed that even in the reaction between an amine and an aldehyde, the mechanochemical method can advantageously synthesize a polymer compound rather than the reaction in a liquid phase.

## Claims

1. A method for producing a block copolymer, the method comprising a step of mechanochemically treating a mixture containing a protein and a molecule capable of plasticizing a protein.

2. The method according to claim 1, wherein the block copolymer contains a protein and a molecule capable of plasticizing a protein as unit structures.

3. The method according to claim 1, further comprising, after the step of performing the mechanochemical treatment, a step of freeze-drying the mechanochemically treated product.

4. The method according to claim 1, wherein the number of reactive functional groups contained in the molecule capable of plasticizing a protein is larger than the number of reactive functional groups contained in the protein.

5. The method according to claim 4, wherein the use amount of the molecule capable of plasticizing a protein is larger than the use amount of the protein based on the number of moles.

6. The method according to claim 4, wherein the reactive functional group in the protein is a nucleophilic functional group, and the reactive functional group in the molecule capable of plasticizing a protein is an electrophilic functional group.

7. The method according to claim 6, wherein the nucleophilic functional group is a thiol group, and the electrophilic functional group is a thiol-reactive group.

8. The method according to claim 6, wherein the nucleophilic functional group is an amino group, and the electrophilic functional group is an amine-reactive group.

9. The method according to claim 1, wherein the protein contains a hydrophobic protein.

10. The method according to claim 9, wherein the hydrophobic protein has a hydropathy index of more than 0.

11. The method according to claim 1, wherein the protein contains an artificial protein.

12. The method according to claim 11, wherein the artificial protein contains an artificial structural protein.

13. The method according to claim 1, wherein the mechanochemical treatment is implemented by applying a shear force to the mixture.

14. The method according to claim 13, wherein the shear force is applied to the mixture using a mixer mill or an extruder.

15. A method for producing an aqueous dispersion of a block copolymer, the method comprising a step of dispersing a block copolymer obtained by the method according to any one of claims 1 to 12 in an aqueous medium.

16. A method for producing a water-dispersible adhesive, the method comprising a step of dispersing a block copolymer obtained by the method according to any one of claims 1 to 12 in an aqueous medium.

17. A method for producing a coating liquid, the method comprising a step of dispersing a block copolymer obtained by the method according to any one of claims 1 to 12 in an aqueous medium.

18. A method for producing a molded body, the method comprising a step of molding a block copolymer obtained by the method according to any one of claims 1 to 12.

19. The method according to claim 18, wherein the molding step is a step of subjecting the block copolymer to heat-and-pressure molding.

20. A method for producing a solution-state adhesive, the method comprising a step of dissolving a block copolymer obtained by the method according to any one of claims 1 to 12 in a solvent.

21. A method for producing a film-shaped adhesive, the method comprising a step of molding a film from a solution in which a block copolymer obtained by the method according to any one of claims 1 to 12 is dissolved.

22. A method for producing a powdery adhesive, the method comprising a step of obtaining a powder composition containing a block copolymer obtained by the method according to any one of claims 1 to 12.

23. A method for producing an adhesive body, the method comprising interposing a solution obtained by dissolving a block copolymer obtained by the method according to any one of claims 1 to 12 in a solvent between a plurality of adherends, and then removing the solvent from the solution to solidify the block copolymer, thereby bonding the adherends to each other.

24. A method for producing an adhesive body, the method comprising interposing an aqueous dispersion obtained by dispersing a block copolymer obtained by the method according to any one of claims 1 to 12 in an aqueous medium between a plurality of adherends, and then removing the aqueous medium from the aqueous dispersion to solidify the block copolymer, thereby bonding the adherends to each other.

25. A method for producing an adhesive body, the method comprising softening a film containing a block copolymer obtained by the method according to any one of claims 1 to 12 by swelling or heating, interposing the film between a plurality of adherends, and then curing the film in a state of being brought into pressure contact with the adherends, thereby bonding the adherends to each other.

26. A method for producing an adhesive body, the method comprising heating a powder composition containing a block copolymer obtained by the method according to any one of claims 1 to 12 in a state where the powder composition is interposed between a plurality of adherends, and pressurizing the powder composition via the adherends to solidify the powder composition, thereby bonding the adherends to each other.

27. A method for producing a solution-state coating agent, the method comprising a step of dissolving a block copolymer obtained by the method according to any one of claims 1 to 12 in a solvent.

28. A method for producing a water-dispersible coating agent, the method comprising a step of dispersing a block copolymer obtained by the method according to any one of claims 1 to 12 in an aqueous medium.

29. A method for producing a film-shaped coating agent, the method comprising a step of molding a film from a solution in which a block copolymer obtained by the method according to any one of claims 1 to 12 is dissolved.

30. A method for producing a powdery coating agent, the method comprising a step of obtaining a powder composition containing a block copolymer obtained by the method according to any one of claims 1 to 12.

31. A method for producing a laminate including a base material and a coating layer laminated on at least a part of a surface of the base material, the method comprising
supplying a solution obtained by dissolving a block copolymer obtained by the method according to any one of claims 1 to 12 in a solvent to at least a part of the surface of the base material to coat at least a part of the surface of the base material with the solution, and then removing the solvent from the solution to solidify the block copolymer, thereby laminating the coating layer on at least a part of the surface of the base material.

32. A method for producing a laminate including a base material and a coating layer laminated on at least a part of a surface of the base material, the method comprising
supplying an aqueous dispersion obtained by dispersing a block copolymer obtained by the method according to any one of claims 1 to 12 in an aqueous medium to at least a part of the surface of the base material to coat at least a part of the surface of the base material with the aqueous dispersion, and then removing the aqueous medium from the aqueous dispersion to solidify the block copolymer, thereby laminating the coating layer on at least a part of the surface of the base material.

33. A method for producing a laminate including a base material and a coating layer laminated on at least a part of a surface of the base material, the method comprising
softening a film containing a block copolymer obtained by the method according to any one of claims 1 to 12 by swelling or heating, placing the film on at least a part of the surface of the base material, and then curing the film in a state of being brought into pressure contact with the base material, thereby laminating the coating layer on at least a part of the surface of the base material.

34. A method for producing a laminate including a base material and a coating layer laminated on at least a part of a surface of the base material, the method comprising
heating a powder composition containing a block copolymer obtained by the method according to any one of claims 1 to 12 in a state where the powder composition is placed on at least a part of the surface of the base material, and pressurizing the powder composition between a pressurizing body and the base material to solidify the powder composition, thereby laminating the coating layer on at least a part of the surface of the base material.
